# EUROPEAN PATENT SPECIFICATION

(11) **EP 3 675 810 B1**
(45) Date of publication and mention of the grant of the patent: **14.10.2026**
(21) Application number: 18758639.1
(22) Date of filing: 29.08.2018
(51) Int. Cl.: A61K 8/60, A61K 8/73, A61Q 5/12, A61Q 5/00, A45D 2/00, A45D 7/06

(54) **PROCESS FOR TREATING KERATIN FIBERS USING A MONOSACCHARIDE WITH AMINE GROUP, A POLYSACCHARIDE WITH AMINE GROUP AND A STEP OF HEAT TREATMENT**
VERFAHREN ZUR BEHANDLUNG KERATINISCHER FASERN UNTER VERWENDUNG EINES MONOSACCHARIDS MIT EINER AMINGRUPPE, EINEM POLYSACCHARID MIT AMINGRUPPE UND EINEM WÄRMEBEHANDLUNGSSCHRITT
PROCÉDÉ DE TRAITEMENT DE FIBRES DE KÉRATINE UTILISANT UN MONOSACCHARIDE AVEC GROUPE AMINE, UN POLYSACCHARIDE AVEC GROUPE AMINE ET UNE ÉTAPE DE TRAITEMENT THERMIQUE

(30) Priority: 31.08.2017 FR 1758048
(43) Date of publication of application: 08.07.2020
(73) Proprietor: L'OREAL, 75008 Paris (FR)
(72) Inventor: GREAVES, Andrew, 93601 Aulnay-sous-Bois (FR); BAGHDADLI, Nawel, 93601 Aulnay-sous-Bois (FR); BARIL, Bérangère, 93601 Aulnay-sous-Bois (FR); BILDSTEIN, Lucien, 93601 Aulnay-sous-Bois (FR)
(74) Representative: L'Oreal
(86) International application number: PCT/EP2018/073183
(87) International publication number: WO 2019/043032

(56) References cited:
- EP-A1- 0 414 608
- WO-A1-2016/142551
- WO-A2-02/078649
- FR-A1- 3 033 498
- JP-A- 2000 281 540
- US-A- 4 542 014
- DATABASE GNPD [online] MINTEL; 28 February 2004 (2004-02-28), ANONYMOUS: "Hair relaxing balm", XP002780279, retrieved from www.gnpd.com Database accession no. 10160754
- ONSOYEN E ET AL: "ADDING BENEFITS TO COSMETIC FORMULATIONS BY TAILORMADE CHITOSANS", SOFW JOURNAL,, vol. 117, no. 16, 24 October 1991 (1991-10-24), pages 633 - 637, XP000267861, ISSN: 0942-7694

## Description

The invention relates to i) a process for treating keratin fibres, in particular human keratin fibres such as the hair, employing a) at least one monosaccharide with amine group, b) at least one polysaccharide with amine group(s) then a heat treatment or heat (iii); ii) a composition having an acid pH **(C)** comprising the ingredients a) and b) in salified or non-salified form; iii) the use of the ingredients a) and b) then of heat (iii) to treat the keratin fibres, in particular human keratin fibres such as the hair, and iv) a multi-compartment kit or device comprising a) and b).

Hair is generally damaged and weakened by the action of external atmospheric agents such as light, sun and bad weather, and also by mechanical or chemical treatments, such as brushing, combing, dyeing, bleaching, permanent-waving, relaxing and repeated washing. Hair is thus damaged by these various factors and may in the long run become dry, coarse, brittle, dull, split and/or limp.

Thus, to overcome these drawbacks, it is common practice to resort to hair treatments which make use of compositions intended for conditioning the hair appropriately by giving it satisfactory cosmetic properties, especially smoothness, sheen, a soft feel (a natural feel; the hair is no longer coarse), suppleness, a lightweight feel, good disentangling properties leading to easy combing, and good manageability of the hair which is thus easy to shape.

These hair care compositions can, for example, be conditioning shampoos, hair conditioners, masks or serums. However, the conditioning effect obtained fades out in the course of successive shampoo washes and does not show satisfactory persistence on shampooing.

It is known practice to employ care compositions comprising reducing sugars such as monosaccharides, used as conditioning agents, especially to repair keratin fibres which have been damaged by harsh treatments.

Indeed, patent application US 2002/0193264 describes a process for conditioning keratin fibres, in which at least one sugar chosen from monosaccharides is applied to said fibres, and a step of heating the keratin fibres is carried out. Similarly, patent application US 2002/0172653 discloses a process for conditioning keratin fibres comprising a step of applying to said fibres a sugar chosen from specific C₅-C₇ monosaccharides and a step of heating the keratin fibres. However, the use of reducing sugars followed by a heat treatment may lead to an undesired modification of the colour of the keratin fibres. Thus, the keratin fibres are not always sufficiently protected, repaired or cosmetically transformed in a long-lasting manner. Another document describes the use of oxidized polysaccharide with an amine-containing monosaccharide (WO 2016/142551). Nevertheless, the results obtained are not always satisfactory in terms of repairing or preventing breakage of the keratin fibres, in particular split keratin fibres, when a treatment with heat is carried out.

There is therefore a real need to develop a composition and a process for treating keratin fibres, in particular human keratin fibres such as the hair, which is able to protect and/or repair said fibres, if possible in a lasting manner, with respect to external agents and/or heat.

Moreover, it is advantageous to find a means for treating damaged keratin fibres by repairing them, that is to say by improving the cosmetic effect of damaged keratin fibres intrinsically, while retaining or even improving the manageability, the anti-frizz properties and/or the persistence of the styling of said fibres. It is also advantageous to prevent the breakage of natural keratin fibres, while retaining or even improving the manageability, the anti-frizz properties and/or the persistence of the styling of said fibres.

This (these) technical problem(s) is (are) solved by the process for treating keratin fibres, especially human keratin fibres, in particular the hair, comprising:
(i) a step consisting in applying, to said fibres, a) one or more monosaccharides with amine group;
(ii) a step consisting in applying, to said fibres, b) one or more polysaccharides with amine group(s);
(ii') optionally a drying step;
(iii) then a step of heat treatment at a temperature above or equal to 80°C, in particular at a temperature between 100°C and 250°C, preferably with a hair iron;

it being understood that the steps (i) and (ii) may be carried out simultaneously, or sequentially, preferably the steps (i) and (ii) are carried out simultaneously and that the drying step (ii'), when it is present, precedes the heat treatment step and follows the steps (i) and (ii);
in which a) the monosaccharide(s) with amine group is (are) in a cosmetic composition having an acid pH, i.e. pH below 7; and
in which b) the polysaccharide(s) with amine group(s) is (are) in a cosmetic composition having an acid pH, i.e. pH below 7; and
in which a) the monosaccharide(s) with amine group is (are) chosen from the compounds of formula **(A')** below and also the organic or mineral acid salts thereof, preferably mineral acid salts thereof, and also the α or β anomers thereof, the optical isomers thereof of L or D configuration, preferably D configuration, and the solvates thereof such as hydrates: 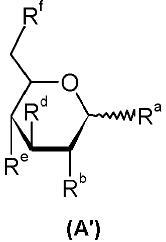
in which formula **(A') R^{b}** represents NH₂, and **R^{a}**, **R^{d}**, **R^{e}** and **R^{f}** represent a hydroxyl group; and
in which b) the polysaccharide(s) with amine group(s) is (are) of formula **(B₃)** below, and also the organic or mineral acid salts thereof, and the solvates thereof such as hydrates: 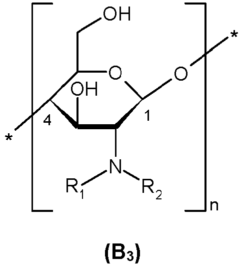
in which formula **(B₃)**:
   - **R₁** and **R₂** represent i) a hydrogen atom or ii) -C(O)-R'₁ with R'₁ representing a (C₁-C₄)alkyl group ; and
   - **n** is an integer greater than or equal to 2, particularly between 3 and 3000 inclusive, more particularly between 5 and 2500, preferentially between 10 and 2300;
it being understood that in the polysaccharide at least one saccharide unit bears an NH₂ amino group and at least one other saccharide unit bears an N(H)-R' group with R' representing a (C₁-C₄)alkylcarbonyl group such as acetyl CH₃-C(O)-.

Another subject of the invention is an aqueous composition having an acid pH (pH < 7), composition also referred to as **(C),** comprising:
either
a) one or more salified monosaccharides with amine group as defined previously; and b) one or more salified polysaccharides with amine group(s) as defined previously, preferably having an average molecular weight of less than or equal to 400 kDa, and optionally c) one more mineral or organic acids, preferably different from the following acids: aspartic acid, glutamic acid, gluconic acid, pyrrolidonecarboxylic acid, 100 OE and 500 OE ethoxylated stearic acid, linoleic acid, succinic acid;
or
a) one or more non-salified monosaccharides with amine group as defined previously; and
b) one or more salified polysaccharides with amine group(s) as defined previously, preferably having an average molecular weight of less than or equal to 400 kDa, and
and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid and alginic acid;
or
a) one or more salified monosaccharides with amine group as defined previously; and
b) one or more non-salified polysaccharides with amine group(s) as defined previously, preferably having an average molecular weight of less than or equal to 400 kDa,
and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid and alginic acid;
or
a) one or more non-salified monosaccharide(s) with amine group as defined previously;
b) one or more non-salified polysaccharide(s) with amine group(s) as defined previously, preferably having an average molecular weight of less than or equal to 400 kDa, and
c) one or more mineral or organic acids different from the following acids: aspartic acid, glutamic acid, gluconic acid, pyrrolidonecarboxylic acid, 100 OE and 500 OE ethoxylated stearic acid, linoleic acid, succinic acid and alginic acid.

Another subject of the invention is the use of the ingredients a), b) and optionally c) as defined previously, in which the ingredient(s) a) is (are) in a cosmetic composition having an acid pH, i.e. pH below 7 and the ingredient(s) b) is (are) in a cosmetic composition having an acid pH, i.e. pH below 7, to improve the cosmetic effect of the keratin fibres intrinsically, to prevent or reduce the breakage of natural keratin fibres and/or to repair damaged keratin fibres, while retaining or even improving the manageability, anti-frizz properties and/or the persistence of the styling of said fibres, in particular human fibres such as the hair, when a fibre heating step (iii) is carried out at at least 80°C, in particular at a temperature between 100°C and 250°C, preferably with a hair iron.

Another subject is the use of the composition having an acid pH **(C)** as defined previously to improve the cosmetic effect of the keratin fibres intrinsically, to prevent or reduce the breakage of natural keratin fibres and/or to repair damaged keratin fibres, while retaining or even improving the manageability, anti-frizz properties and/or the persistence of the styling of said fibres, in particular human fibres such as the hair, when a fibre heating step is carried out, especially at a temperature above 80°C, in particular between 80°C and 250°C, preferably with a hair iron.

Another subject of the invention is a cosmetic treatment process using the composition having an acid pH **(C)** as described previously to improve the cosmetic effect of the keratin fibres intrinsically, to prevent or reduce the breakage of natural keratin fibres and/or to repair damaged keratin fibres, while retaining or even improving the manageability, anti-frizz properties and/or the persistence of the styling of said fibres, in particular human fibres such as the hair, when a fibre heating step is carried out, especially at a temperature above 80°C, in particular between 80°C and 250°C, preferably with a hair iron.

Another subject of the invention is a process for treating keratin fibres, especially human keratin fibres, in particular the hair, comprising:
(i) a step consisting in applying, to said fibres, a cosmetic composition **(ca)** containing a) one or more monosaccharides with amine group as defined previously;
(ii) a step consisting in applying, to said fibres, a cosmetic composition **(cb)** containing b) one or more polysaccharides with amine group(s) as defined previously, preferably having an average molecular weight of less than or equal to 400 kDa;
(ii') optionally a drying step;
(iii) then a step of heat treatment at a temperature above or equal to 80°C, in particular at a temperature between 100°C and 250°C, preferably with a hair iron;
it being understood that the steps (i) and (ii) may be carried out simultaneously, or sequentially, preferably the steps (i) and (ii) are carried out simultaneously and that the drying step (ii'), when it is present, precedes the heat treatment step and follows the steps (i) and (ii).

In particular, hair treated with the method according to the invention remains well-behaved since the presence of frizziness is not observed. Thus, the hairs are aligned, smooth and disentangle easily, which makes them easier to comb. The treated hair also has more body (it is not limp) and is thus easier to style. The treated hair is easy to shape, this shaping of the hair persisting even after several shampoo washes. Moreover, the treated hair is also shiny and soft to the touch. It is stronger and less brittle. The process according to the invention has the advantage of giving good persistence of these good hair-conditioning cosmetic properties after at least one shampoo wash, in particular after 5 successive shampoo washes. Thus, the treated hair is conditioned in a long-lasting manner. After treatment, the hair is not laden, and has a natural feel. The process, the composition **(C)** and the use of the invention make it possible to repair the split ends of keratin fibres, especially human keratin fibres such as the hair. Moreover, the process, the composition **(C)** and the use of the invention make it possible to protect the synthetic (oxidation and direct) dyeing of the keratin fibres, to reduce the fading of the colour of said fibres linked in particular to successive shampoo washes, and/or to repeated hair iron treatments, especially straightening iron treatments, without damaging said fibres.

In the following text, unless indicated otherwise:
▪ the term *"monosaccharides"* is understood to mean a monosaccharide sugar comprising at least 5 carbon atoms of formula Cₓ(H₂O)ₓ with x an integer greater than or equal to 5, preferably x is greater than or equal to 6, in particular x is between 5 and 7 inclusive, preferably x = 6, they may be of D or L configuration, and of alpha or beta anomer, and also the salts thereof and the solvates thereof such as hydrates;
▪ the term *"polysaccharides"* is understood to mean a polysaccharide sugar which is a polymer constituted of several saccharides bonded to one another by O-glucosidic bonds, said polymers being constituted of monosaccharide units as defined previously, said monosaccharide units comprising at least 5 carbon atoms, preferably 6, particularly the monosaccharide units are connected to one another by 1,4 or 1,6 bond as α (alpha) or β (beta) anomer, it being possible for each saccharide unit to be of L or D configuration, and also the salts thereof and the solvates thereof such as the hydrates of said monosaccharides; more particularly they are polymers formed of a certain number of saccharides (or monosaccharides) having the general formula: -[Cₓ(H₂O)_{y})]ₙ- where x is an integer greater than or equal to 5, preferably x is greater than or equal to 6, in particular x is between 5 and 7 inclusive and preferably x = 6, and y is an integer which represents x - 1, and n is an integer greater than or equal to 2, particularly of between 3 and 3000 inclusive, more particularly between 5 and 2500 and preferentially between 10 and 2300;
▪ the term *"with amine group(s)"* is understood to mean that the monosaccharide(s) a) and the polysaccharide(s) b) is (are) substituted with one or more NR₁R₂ amino group(s) i.e. at least one of the hydroxyl groups of at least one saccharide unit is replaced with an NR₁R₂ group with R₁ and R₂, which are identical or different, representing i) a hydrogen atom, ii) a (C₁-C₆)alkyl group that is optionally substituted, preferably with one or more hydroxyl or NH₂ groups, iii) an aryl group such as phenyl, iv) an aryl(C₁-C₄)alkyl group such as benzyl, v) a (hetero)cyclo(C₅-C₇)alkyl group such as cyclohexyl, morpholinyl, piperazinyl, piperidinyl, vi) a (hetero)cyclo(C₅-C₇)alkyl(C₁-C₄)alkyl group such as cyclohexylmethyl, vii) -C(Y)-(Y')ₚ-R'₁ with Y and Y', which are identical or different, representing an oxygen atom, sulfur atom or N(R'₂), preferably oxygen, p = 0 or 1, preferably 0; and R'₁ and R'₂ representing i) to vi) of R₁ and R₂ defined previously, and in particular R'₁ denoting a (C₁-C₆)alkyl group such as methyl. Preferably R₁ and R₂ represent a hydrogen atom or a (C₁-C₄)alkylcarbonyl group such as acetyl;
▪ the term *"low molecular weight"* is understood to mean that the polysaccharide with amine group has an average molecular weight of less than 400 000 Da;
▪ the term *"organic or mineral acid salt"* is more particularly understood to mean organic or mineral acid salts in particular chosen from a salt derived from i) hydrochloric acid HCl, ii) hydrobromic acid HBr, iii) sulfuric acid H₂SO₄, iv) alkylsulfonic acids: Alk-S(O)₂OH such as methylsulfonic acid and ethylsulfonic acid; v) arylsulfonic acids: Ar-S(O)₂OH such as benzenesulfonic acid and toluenesulfonic acid; vi) alkoxysulfinic acids: Alk-O-S(O)OH such as methoxysulfinic acid and ethoxysulfinic acid; vii) aryloxysulfinic acids such as tolueneoxysulfinic acid and phenoxysulfinic acid; viii) phosphoric acid H₃PO₄; ix) triflic acid CF₃SO₃H and x) tetrafluoroboric acid HBF₄; xi) organic monocarboxylic acids **(I) R-C(O)-OH** with **R** representing a (hetero)aryl group such as phenyl, a (hetero)aryl(C₁-C₄)alkyl such as benzyl, or (C₁-C₃₀)alkyl or an unsaturated C₂-C₃₀ radical (i.e. comprising at least one ethylenic unsaturation, preferably one ethylenic unsaturation) said alkyl group or unsaturated C₂-C₃₀ radical being optionally interrupted and/or optionally substituted preferably with one or more hydroxyl groups and not substituted with one or more amino radicals, R preferably denoting a (C₁-C₆)alkyl group optionally interrupted and/or optionally substituted with 1, 2 or 3 hydroxyl groups, preferably R represents a (C₁-C₄)alkyl group such as methyl or ethyl; in particular the organic monocarboxylic acids **(I)** are chosen from acetic acid, glycolic acid and lactic acid, more particularly from acetic acid and lactic acid, and xii) the polycarboxylic acids of formula **(II)** below: 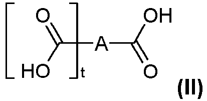 in which formula **(II):**
   **A** represents a saturated or unsaturated, cyclic or noncyclic, aromatic or nonaromatic, polyvalent hydrocarbon-based group comprising from 1 to 30 carbon atoms optionally interrupted with one or more heteroatoms such as oxygen and/or optionally substituted in particular with one or more hydroxyl groups and t represents an integer between 1 and 5 inclusive; preferably A represents a divalent (C₁-C₆)alkylene group optionally substituted in particular with one or more hydroxyl groups and not substituted with at least one amino radical, and t is equal to 1, 2 or 3, the polycarboxylic acids of formula **(II)** are preferably chosen from tartaric acid, succinic acid, fumaric acid and citric acid and more particularly chosen from tartaric acid, succinic acid, fumaric acid, maleic acid and citric acid; and xiii) amino acids comprising more carboxylic acid radicals than amino groups such as gamma-carboxyglutamic acid, aspartic acid, glutamic acid, in particular gamma-carboxyglutamic acid; in particular the salts of monocarboxylic acids different from pyrrolidonecarboxylic acid, 100 OE and 500 OE ethoxylated stearic acid and linoleic acid, more particularly of monocarboxylic acid of formula ;
▪ an *"alkyl radical"* is a linear or branched C₁-C₁₀, preferably C₁-C₆ and in particular C₁-C₄ hydrocarbon-based radical, such as methyl or ethyl, unless otherwise indicated;
▪ a (Cₓ-C_{y})alkyl radical is a linear or branched Cₓ-C_{y} hydrocarbon-based radical;
▪ the expression *"optionally interrupted"* attributed to the alkyl radical or to the polyvalent group A defined previously is understood to mean that said radical may be interrupted with one or more groups or heteroatoms chosen from O, S, CO or combinations thereof such as -CO-O- or -O-CO-, preferably interrupted with one or more non-adjacent oxygen atoms;
▪ the expression *"optionally substituted"* attributed to the alkyl radical or to the polyvalent group A defined previously is understood to mean that said radical may be substituted with one or more radicals chosen from the following radicals i) hydroxyl, ii) C₁-C₄ alkoxy, iii) acylamino, iv) amino optionally substituted with one or two identical or different C₁-C₄ alkyl radicals, said alkyl radicals possibly forming, with the nitrogen atom that bears them, a 5- to 7-membered heterocycle, optionally comprising another nitrogen or non-nitrogen heteroatom;
▪ an *"alkoxy radical"* is an alkyl-oxy radical for which the alkyl radical is a linear or branched C₁-C₁₀, preferentially C₁-C₆ and more particularly C₁-C₄ hydrocarbon-based radical such as methoxy or ethoxy;
▪ when the alkoxy group is optionally substituted, this implies that the alkyl group is optionally substituted as defined hereinabove;
▪ the *"aryl"* or *"heteroaryl"* radicals or the aryl or heteroaryl part of a radical may be substituted with at least one substituent borne by a carbon atom, chosen from one of the following atoms or groups:
   - halogen;
   - optionally substituted C₁-C₆, preferably C₁-C₄, alkyl;
   - hydroxyl;
   - C₁-C₂ alkoxy;
   - C₁-C₄ (poly)hydroxyalkoxy;
   - amino;
   - 5- or 6-membered heterocycloalkyl;
   - 5- or 6-membered heteroaryl, optionally substituted with a (C₁-C₄)alkyl radical, preferentially methyl;
   - amino substituted with one or two optionally substituted, identical or different C₁-C₆ alkyl radicals:
   - acylamino (-NR-C(O)-R') in which the R radical is a hydrogen atom or a C₁-C₄ alkyl radical optionally bearing at least one hydroxyl group and the R' radical is a C₁-C₂ alkyl radical;
   - carbamoyl ((R)₂N-C(O)-) in which the R radicals, which may be identical or different, represent a hydrogen atom or a C₁-C₄ alkyl radical optionally bearing at least one hydroxyl group;
   - alkylsulfonylamino (R'-S(O)₂-N(R)-) in which the R radical represents a hydrogen atom or a C₁-C₄ alkyl radical optionally bearing at least one hydroxyl group and the radical R' represents a C₁-C₄ alkyl radical, or a phenyl radical;
   - aminosulfonyl ((R)₂N-S(O)₂-) in which the R radicals, which may be identical or different, represent a hydrogen atom or a C₁-C₄ alkyl radical optionally bearing at least one hydroxyl group;
   - cyano;
   - nitro or nitroso; and
   - polyhaloalkyl, preferentially trifluoromethyl;
   the cyclic or heterocyclic part of a non-aromatic group may be substituted with at least one substituent chosen from the following groups:
   - hydroxyl;
   - C₁-C₄ alkoxy or C₂-C₄ (poly)hydroxyalkoxy;
   - C₁-C₄ alkyl;
   - alkylcarbonylamino (R-C(O)-N(R')-), in which the R' radical is a hydrogen atom or a C₁-C₄ alkyl radical optionally bearing at least one hydroxyl group and the R radical is a C₁-C₂ alkyl group or an amino optionally substituted with one or two identical or different C₁-C₄ alkyl groups themselves optionally bearing at least one hydroxyl group;
   - alkylcarbonyloxy (R-C(O)-O-), in which the R radical is a C₁-C₄ alkyl radical or an amino group optionally substituted with one or two identical or different C₁-C₄ alkyl groups themselves optionally bearing at least one hydroxyl group;
   - alkoxycarbonyl (R-G-C(O)-), in which the R radical is a C₁-C₄ alkoxy radical and G is an oxygen atom, or an amino group optionally substituted with a C₁-C₄ alkyl group itself optionally bearing at least one hydroxyl group;
▪ a cyclic or heterocyclic radical, or a non-aromatic part of an aryl or heteroaryl radical, may also be substituted with one or more oxo groups;
▪ an *"aryl"* radical represents a monocyclic or fused or non-fused polycyclic carbon-based group, comprising from 6 to 22 carbon atoms, at least one ring of which is aromatic; such as phenyl, biphenyl, naphthyl, indenyl, anthracenyl or tetrahydronaphthyl, preferentially phenyl;
▪ a *"heteroaryl radical"* represents an optionally cationic, 5- to 22-membered, monocyclic or fused or non-fused polycyclic group, comprising from 1 to 6 heteroatoms chosen from nitrogen, oxygen, sulfur and selenium atoms, at least one ring of which is aromatic; preferentially, a heteroaryl radical is chosen from acridinyl, (benz)imidazolyl, (benzo)bistriazolyl, (benzo)pyrazolyl, (benzo)pyridazinyl, (benzo)quinolyl, (benzo)thiazolyl, (benzo)triazolyl, (benz)oxazolyl, pyridyl, tetrazolyl, dihydrothiazolyl, imidazopyridyl, indolyl, isoquinolyl, naphthoimidazolyl, naphthoxazolyl, naphthopyrazolyl, oxadiazolyl, oxazolyl, oxazolopyridyl, phenazinyl, phenoxazolyl, pyrilyl, pyrazoyltriazyl, pyridyl, pyridinoimidazolyl, pyrrolyl, quinolyl, tetrazolyl, thiadiazolyl, thiazolopyridyl, thiazoylimidazolyl, thiopyrylyl, triazolyl and xanthyl;
▪ a *"heterocyclic radical"* is a 5- to 22-membered monocyclic or fused or non-fused polycyclic radical comprising one or two unsaturations but is non-aromatic, comprising from 1 to 6 heteroatoms chosen from nitrogen, oxygen, sulfur and selenium atoms;
▪ a *"heterocycloalkyl radical"* is a heterocyclic radical comprising at least one saturated ring;
▪ *"limp"* keratin fibres or *"limp"* hair is understood to mean that said fibres or hair are elastic, have no body, do not hold shape, the head of hair is flat without volume;
▪ the expression *"at least one"* is equivalent to *"one or more";* and
▪ the expression *"inclusive"* for a range of concentrations means that the limits of the range are included in the defined interval.

### a) Monosaccharides with amine group

The first ingredient used in the present invention is a) one or more monosaccharides with amine group, and also the organic or mineral acid salts thereof, the α or β anomers thereof, the optical isomers thereof of L or D configuration, and the solvates thereof such as hydrates.

Preferably the monosaccharide(s) with amine group of the invention a) is (are) salified with the aid of a mineral acid such as hydrochloric acid.

According to one particular form of the invention, the monosaccharide(s) with amine group a) of the invention denote(s) a mixture of monosaccharide(s) with amine group, one of which is glucosamine or the salts thereof of an organic or mineral acid such as hydrochloric acid, the α or β anomers thereof, the optical isomers thereof of L or D configuration, and the solvates thereof such as hydrates.

The monosaccharide(s) with amine group a) of the invention are chosen from the compounds of formula **(A')** below and also the salts thereof of an organic or mineral acid, preferably a mineral acid such as hydrochloric acid, and also the α or β anomers thereof, the optical isomers thereof of L or D configuration, preferably D configuration, and the solvates thereof such as hydrates: 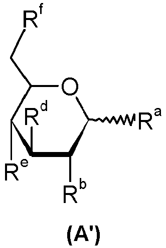 in which formula **(A') R^{b}** represents NH₂, and **R^{a}, R^{d}, R^{e}** and **R^{f}** represent a hydroxyl group.

According to one preferred embodiment, the monosaccharide(s) with amine group of the invention a) is (are) present in the composition (C) or in the composition (ca) in an amount of between 0.01% and 20% by weight relative to the total weight of the composition which comprises same, more particularly between 0.05% and 10%, more preferentially between 0.1% and 5% by weight relative to the total weight of the composition which comprises same.

### b) Polysaccharides with amine qroup(s)

The second ingredient used in the present invention is b) one or more polysaccharides with amine group(s), and also the organic or mineral acid salts thereof, the α or β anomers thereof, the optical isomers thereof of L or D configuration, and the solvates thereof such as hydrates.

According to one preferred embodiment of the invention, the polysaccharide(s) with amine group(s) b) has (have) an average molecular weight MW of less than or equal to 400 kDa, particularly less than 200 kDa. According to one particular embodiment, the polysaccharide(s) with amine group(s) b) have a low average molecular weight MW i.e. have an MW < 100 kDa, preferentially have an average MW which is < 40 kDa, more preferentially have an average MW which is between 1 kDa and 30 kDa inclusive, more preferentially still have an average MW which is between 3 kDa and 28 kDa inclusive.

The polysaccharide(s) with amine group(s) b) of the invention is (are) of natural animal or plant origin, or else are derived from synthesis, hemisynthesis or biosynthesis.

The polysaccharide(s) with amine group(s) of the invention b) is (are) chosen from those of formula **(B₃)** below, and also the organic or mineral acid salts thereof, and the solvates thereof such as hydrates: 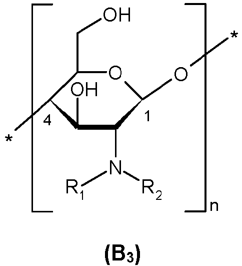 in which formula **(B₃):**
- **R₁** and **R₂** represent i) a hydrogen atom or ii) -C(O)-R'₁ with R'₁ representing a (C₁-C₄)alkyl group such as methyl; and
- **n** is an integer greater than or equal to 2, particularly between 3 and 3000 inclusive, more particularly between 5 and 2500, preferentially between 10 and 2300;
it being understood that in the polysaccharide b) of formula (**B₃**) at least one saccharide unit bears an NH₂ amino group and at least one other saccharide unit bears an N(H)-R' group with R' representing a (C₁-C₄)alkylcarbonyl group such as acetyl CH₃-C(O)-.

More particularly, the polysaccharide(s) with amine group(s) of the invention b) is (are) chosen from chitosans of formula **(B₄)** below, and also the organic or mineral acid salts thereof, and the solvates thereof such as hydrates: 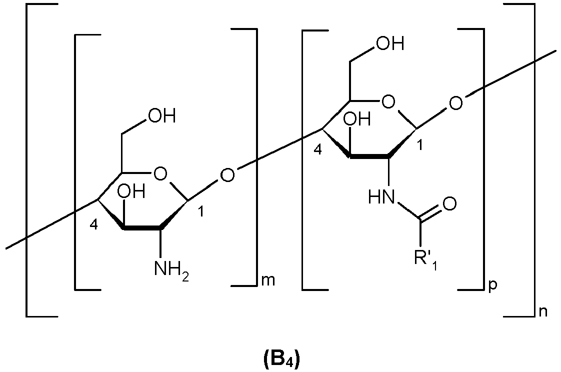 in which formula **(B₄):**
▪ **R'₁** representing a (C₁-C₄)alkyl group such as methyl; and
▪ **n** is an integer greater than or equal to 2, particularly between 3 and 3000 inclusive, more particularly between 5 and 2500, preferentially between 10 and 2300;
▪ **P** is greater than 0 and ranges up to 0.5, preferably from 0.05 to 0.3, and better still from 0.1 to 0.20 such as 0.15 with m+p being equal to 1;
it being understood that in the chitosan at least one saccharide unit bears an NH₂ amino group and at least one other saccharide unit bears an N(H)-R', group with R' representing a (C₁-C₄)alkylcarbonyl group such as acetyl CH₃-C(O)-.

For example, when m = 0.7, p = 0.3 this means that 70% of the amine groups are free (unsubstituted) and 30% of the amino groups are N-alkyl(C₁-C₄)carbonyl groups, in particular N-acetyl groups, corresponding to the chitosan of formula: 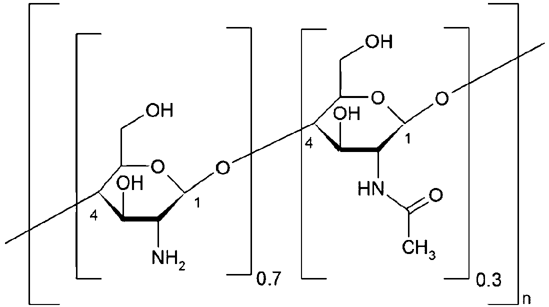 with n as defined above.

Particularly, the polysaccharide(s) with amine group(s) of the invention b) is (are) chosen from chitosans, salified using organic acid, preferentially using monocarboxylic acid of formula **(I)** as defined above or polycarboxylic acid of formula **(II)** as defined above, more preferentially still salified using carboxylic acid of formula **(I)** such as lactic acid.

According to one particular form of the invention, the polysaccharide(s) with amine group(s) b) of the invention denote(s) a mixture of polysaccharide(s) with amine group(s), one of which is a chitosan or the organic or mineral acid salts thereof, preferably the salts thereof of an organic acid such as lactic acid, the α or β anomers thereof, the optical isomers thereof of L or D configuration, and the solvates thereof such as hydrates.

According to another form of the invention, the polysaccharide(s) with amine group(s) b) of the invention denote(s) a single polysaccharide with amine group(s), in particular a mixture of chitosan or the organic or mineral salts thereof or more particularly the organic acid salts thereof such as the lactic acid salt thereof, the α or β anomers thereof, the optical isomers thereof of L or D configuration, and the solvates thereof such as hydrates.

According to another form of the invention, the polysaccharide(s) with amine group(s) b) of the invention denote(s) a single polysaccharide with amine group(s), in particular a chitosan or the organic or mineral acid salts thereof or more particularly the organic acid salts thereof such as the lactic acid salt thereof, the α or β anomers thereof, the optical isomers thereof of L or D configuration, and the solvates thereof such as hydrates.

According to one preferred embodiment, the polysaccharide(s) with amine group(s) of the invention b) of formula **(B₃)** or **(B₄)** as defined above is (are) present in the composition (C) or in the composition **(cb)** in an amount of between 0.01% and 20% by weight relative to the total weight of the composition which comprises same, more particularly between 0.05% and 10%, more preferentially between 0.1% and 5% by weight relative to the total weight of the composition which comprises same.

### The compositions

The composition of the invention (C) and the composition(s) **(ca)** and/or **(cb)** used in the invention or the process of the invention comprising the ingredients a) and/or b) as defined above are cosmetic, i.e. contain a physiologically acceptable medium, that is to say that is compatible with human keratin materials such as the skin (of the body, face, around the eyes or the scalp), the hair, the eyelashes, the eyebrows, body hair, the nails or the lips.

The physiologically acceptable medium of the composition(s) **(C), (ca), (cb)** used in the process according to the invention is advantageously an aqueous medium. It may for example contain water or a mixture of water and at least one cosmetically acceptable organic solvent. Examples of cosmetically acceptable organic solvents that may be mentioned include C₂-C₄ lower alcohols, such as ethanol and isopropanol; polyols, especially those containing from 2 to 6 carbon atoms, for instance glycerol, propylene glycol, butylene glycol, pentylene glycol, hexylene glycol, dipropylene glycol or diethylene glycol; polyol ethers, for instance 2-butoxyethanol, propylene glycol monomethyl ether and diethylene glycol monomethyl ether or monoethyl ether; and mixtures thereof.

Preferably, the cosmetic composition(s) comprise from 50% to 99.8% by weight of water, relative to the weight of the composition(s).

The composition(s) used according to the invention may also contain one or more cosmetic additives usually used in cosmetics chosen from nonionic, anionic, cationic and amphoteric surfactants, vitamins and provitamins, including panthenol, fillers, opacifiers, sequestrants, film-forming polymers, thickeners, antioxidants, antifoams, moisturizers, emollients, penetrants, fragrances and preserving agents.

The composition(s) **(C), (ca), (cb)** used according to the invention may be in any presentation form conventionally used for hair application.

### pH of the compositions:

The pH values of the composition(s) **(C), (ca), (cb)** of the invention may be adjusted with an organic or mineral acid, or with an alkaline agent chosen from mineral, organic or hybrid alkaline agents or mixtures thereof.

The composition **(ca)** which comprises a) one or more monosaccharides with amine group as defined above, in particular glucosamine, is at an acid pH, i.e. a pH below 7, in particular below or equal to 6, more particularly at a pH between 2 and 6 inclusive, preferentially at a pH between 2.5 and 5.5, more preferentially between 3.5 and 5 such as 4.5. According to one preferred embodiment of the invention, the monosaccharide(s) with amine group a) are in the presence of one or more mineral acids, more particularly hydrochloric acid.

The pH values may be adjusted with c) one or more organic or mineral acids as defined above in *"organic or mineral acid salt".*

The composition **(cb)** which comprises b) one or more polysaccharides with amine group(s) of formula **(B₃)** or **(B₄)** as defined above such as chitosan, is at an acid pH, i.e. a pH below 7, in particular below or equal to 6, more particularly at a pH between 2 and 6 inclusive, preferentially at a pH between 2.5 and 5.5, more preferentially between 3.5 and 5 such as 4.5. According to one particular embodiment of the invention, the polysaccharide(s) with amine group(s) b) are in the presence of one or more organic acids, preferentially monocarboxylic acid(s) of formula **(I)** or polycarboxylic acid(s) of formula **(II)** as defined above, more preferentially still one or more carboxylic acids of formula **(I)** such as lactic acid.

According to a particular embodiment of the invention, the composition **(C)** comprises:
- a) one or more monosaccharides with amine group as defined above, in particular glucosamine, in the presence of one or more mineral acids, more particularly hydrochloric acid, and
- b) one or more polysaccharides with amine group(s) of formula **(B₃)** or **(B₄)** as defined above, especially chitosan, in particular chitosan having an average molecular weight of less than or equal to 400 kDa, in the presence of one or more organic acids, more particularly lactic acid;
- and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid,
and is at an acid pH, i.e. a pH below 7, in particular below or equal to 6, more particularly at a pH between 2 and 6 inclusive, preferentially at a pH between 2.5 and 5.5, more preferentially between 3.5 and 5 such as 4.5.

According to another particular embodiment of the invention, the composition **(C)** comprises:
- a) one or more monosaccharides with amine group as defined above, in particular glucosamine, in the absence of mineral or organic acids, and
- b) one or more polysaccharides with amine group(s) of formula **(B₃)** or **(B₄)** as defined above, especially chitosan, in the presence of one or more organic acids, more particularly lactic acid,
- and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid,
and is at an acid pH, i.e. a pH below 7, in particular below or equal to 6, more particularly at a pH between 2 and 6 inclusive, preferentially at a pH between 2.5 and 5.5, more preferentially between 3.5 and 5 such as 4.5.

According to a particular embodiment of the invention, the composition **(C)** comprises:
- a) one or more monosaccharides with amine group as defined above, in particular glucosamine, in the presence of one or more mineral acids, more particularly hydrochloric acid, and
- b) one or more polysaccharides with amine group(s) of formula **(B₃)** or **(B₄)** as defined above, especially chitosan, in particular chitosan having an average molecular weight of less than or equal to 400 kDa, in the absence of organic or mineral acid(s),
- and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid,
and is at an acid pH, i.e. a pH below 7, in particular below or equal to 6, more particularly at a pH between 2 and 6 inclusive, preferentially at a pH between 2.5 and 5.5, more preferentially between 3.5 and 5 such as 4.5.

According to a particular embodiment of the invention, the composition **(C)** comprises:
- a) one or more monosaccharides with amine group as defined above, in particular glucosamine, in the absence of mineral acid(s),
- b) one or more polysaccharides with amine group(s) of formula **(B₃)** or **(B₄)** as defined above, especially chitosan, in particular chitosan having an average molecular weight of less than or equal to 400 kDa, in the absence of organic or mineral acid(s),
- and c) one or more mineral or organic acids different from the following acids: aspartic acid, glutamic acid, gluconic acid, pyrrolidonecarboxylic acid, 100 OE and 500 OE ethoxylated stearic acid, linoleic acid, succinic acid, said acid c) preferably being chosen from hydrochloric acid, organic monocarboxylic acids of formula (I) as defined above and in particular lactic acid, citric acid, polycarboxylic acids of formula (II) as defined above and in particular fumaric or maleic acid,
and is at an acid pH, i.e. a pH below 7, in particular below or equal to 6, more particularly at a pH between 2 and 6 inclusive, preferentially at a pH between 2.5 and 5.5, more preferentially between 3.5 and 5 such as 4.5.

The pH values may also be adjusted with one or more mineral alkaline agents which are preferably chosen from aqueous ammonia, alkali metal carbonates or bicarbonates such as sodium or potassium carbonates and sodium or potassium bicarbonates, sodium hydroxide or potassium hydroxide, or mixtures thereof.

The pH values may also be adjusted with one or more organic alkaline agents which are organic amines i.e they contain at least one substituted or unsubstituted amino group. The organic alkaline agent(s) is (are) more preferentially chosen from organic amines with a pK_{b} at 25°C of less than 12, and preferably of less than 10, even more advantageously of less than 6. It should be noted that it is the pK_{b} corresponding to the function of highest basicity.

The pH values may also be adjusted with one or more hybrid compounds. Hybrid compounds that may be mentioned include the salts of the amines mentioned previously with acids such as carbonic acid or hydrochloric acid.

The organic alkaline agent(s) are chosen, for example, from alkanolamines, oxyethylenated and/or oxypropylenated ethylenediamines, amino acids and the compounds of formula **(III)** below: 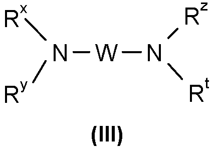 in which formula **(III):**
- **W is** a divalent C₁-C₆ alkylene radical optionally substituted with a hydroxyl group or a C₁-C₆ alkyl radical, and/or optionally interrupted with one or more heteroatoms such as oxygen or NR^{u};
- **R^{x}, R^{y}, R^{z}, R^{t}** and **R^{u},** which may be identical or different, represent a hydrogen atom or a C₁-C₆ alkyl, C₁-C₆ hydroxyalkyl or C₁-C₆ aminoalkyl radical.

### The composition of the invention

According to a particular embodiment of the invention, the composition **(C)** is the composition **(C')** at acid pH and comprises:
a) one or more salified monosaccharides with amine group as defined above;
b) one or more salified polysaccharides with amine group(s) as defined above, preferably having an average molecular weight of less than or equal to 400 kDa,
and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid.

According to another particular embodiment of the invention, the composition **(C)** is the composition **(C")** at acid pH and comprises:
a) one or more non-salified monosaccharides with amine group as defined above; and
b) one or more salified polysaccharides with amine group(s) as defined above, preferably having an average molecular weight of less than or equal to 400 kDa,
and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid.

According to another particular embodiment of the invention, the composition **(C)** is the composition **(C‴)** at acid pH and comprises:
a) one or more salified monosaccharides with amine group as defined above; and
b) one or more non-salified polysaccharides with amine group(s) as defined above, preferably having an average molecular weight of less than or equal to 400 kDa,
and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid.

According to yet another particular embodiment of the invention, the composition **(C)** is the composition **(Cʺʺ)** at acid pH and comprises:
a) one or more non-salified monosaccharides with amine group as defined above;
b) one or more non-salified polysaccharides with amine group(s) as defined above, preferably having a low average molecular weight of less than or equal to 400 kDa, and
c) one or more organic or mineral acids as defined above; preferably different from the following acids: aspartic acid, glutamic acid, gluconic acid, pyrrolidonecarboxylic acid, 100 OE and 500 OE ethoxylated stearic acid, linoleic acid, succinic acid.

### Process for treating keratin fibres

The process for treating keratin fibres, in particular human keratin fibres such as the hair, comprises:
(i) a step consisting in applying, to said fibres, a) one or more monosaccharides with amine group as defined previously;
(ii) a step consisting in applying, to said fibres, b) one or more polysaccharides with amine group(s) as defined previously, preferably having an average molecular weight of less than or equal to 400 kDa;
(ii') optionally a drying step;
(iii) then a step of heat treatment at a temperature above or equal to 80°C, in particular at a temperature between 100°C and 250°C, preferably with a hair iron;
it being understood that the steps (i) and (ii) may be carried out simultaneously, or sequentially, preferably the steps (i) and (ii) are carried out simultaneously and that the drying step (ii'), when it is present, precedes the heat treatment step and follows the steps (i) and (ii).

Another subject of the invention is a process for treating keratin fibres, especially human keratin fibres, in particular the hair, comprising:
(i) a step consisting in applying, to said fibres, a cosmetic composition **(ca)** containing a) one or more monosaccharides with amine group as defined previously ;
(ii) a step consisting in applying, to said fibres, a cosmetic composition **(cb)** containing b) one or more polysaccharides with amine group(s) as defined previously, preferably having an average molecular weight of less than or equal to 400 kDa;
(ii') optionally a drying step;
(iii) then a step of heat treatment at a temperature above or equal to 80°C, in particular at a temperature between 100°C and 250°C, preferably with a hair iron;
it being understood that the steps (i) and (ii) may be carried out simultaneously, or sequentially, preferably the steps (i) and (ii) are carried out simultaneously and that the drying step (ii'), when it is present, precedes the heat treatment step and follows the steps (i) and (ii).

According to a particular embodiment of the process of the invention, steps i) and ii) are carried out simultaneously, or else by applying to the keratin fibres, during a first step, a composition comprising (a) one or more monosaccharides with amine group a) as defined above; and one or more polysaccharides with amine group(s) b) as defined above, i.e.by applying, during a first step, a composition **(C)** as defined above.

According to a particular embodiment of the invention and the composition **(C),** the monosaccharide(s) with amine group a) as defined above; and the polysaccharide(s) with amine group(s) b) as defined above are each found in said composition in an amount of between 0.01% and 20% by weight relative to the total weight of the composition, more particularly between 0.05% and 10%, more preferentially between 0.1% and 5%, more particularly between 0.1% and 5% by weight relative to the total weight of the composition **(C)** comprising same.

The composition **(ca)** applied to said fibres is acidic and comprises one or more organic or mineral acids preferably c) as defined above.

More preferentially, the process of the invention comprises a first step that consists in applying the composition **(C)** as defined above, optionally followed by a drying step, then a step of heat treatment at a temperature above 80°C, in particular at a temperature between 100°C and 250°C, preferably with a hair iron. According to a first advantageous variant, the composition **(C)** is the composition **(C')** as defined above. According to another advantageous variant, the composition **(C)** represents a composition **(C")** as defined above. According to another advantageous variant, the composition **(C)** represents a composition **(C‴)** as defined above. According to yet another advantageous variant, the composition **(C)** represents the composition **(Cʺʺ)** as defined above.

According to a particular variant of the invention, the process of the invention and the use of the invention do not use oxidized polysaccharide, more generally the process of the invention and the use of the invention do not use a compound comprising at least one aldehyde group. According to one preferred embodiment of the invention, the composition(s) **(cb), (C), (C'), (C"), (C‴),** or **(Cʺʺ)** as defined above does not (do not) contain oxidized polysaccharide and more generally the composition(s) **(cb), (C), (C'), (C"), (C‴),** or **(Cʺʺ)** does not (do not) contain a compound comprising at least one aldehyde group.

According to a particular embodiment, the process of the invention is carried out after permanent waving, smoothing, dyeing and/or bleaching of the keratin fibres, in particular human keratin fibres such as the hair.

The process according to invention may also comprise an additional step (ii') of drying the keratin fibres after the application of the monosaccharide(s) with amine group a) as defined above and/or of the polysaccharide(s) with amine group(s) b) as defined above or of the cosmetic compositions containing same and before the step of heating the keratin fibres carried out at a temperature of at least 80°C.

Preferably, the process of the invention carries out a step (ii') of drying the keratin fibres, in particular human keratin fibres such as the hair before the heat treatment step (iii). The drying step is in particular performed using a hairdryer, a hood, or else with a towel, an absorbent or by drying naturally. The drying step is advantageously carried out at a temperature ranging from 20°C to 70°C.

The keratin fibre heat treatment or heating step (iii) of the process of the invention is preferably carried out at a temperature between 80°C and 250°C, it being understood that the temperature is that of the heating means, in particular of the hair iron, and not the temperature of the keratin fibres.

According to one preferred form of the invention, the heat treatment step iii) is carried out on dry keratin fibres.

According to a more particular embodiment of the invention and the process for treating keratin fibres, the fibre heat treatment step or heating step iii) is carried out at a temperature of at least 100°C, in particular at a temperature of between 100°C and 250°C inclusive. Preferably, the step of heating the keratin fibres is carried out at a temperature ranging from 150°C to 220°C, preferably ranging from 160°C to 220°C, preferentially ranging from 160°C to 200°C, especially ranging from 130°C to 190°C. It is understood that the heating temperatures are the temperatures of the heating means, in particular of the hair iron when a hair iron is used, and not the temperature of the keratin fibres. This heat treatment or heating step (iii) is advantageously carried out using a hair iron.

For the purposes of the present invention, the term "hair iron" means a device for heating keratin fibres by placing said fibres in contact with the heating device.

The end of the hair iron which comes into contact with the keratin fibres generally has two surfaces. These two surfaces may be made of metal or of ceramic. In particular, these two surfaces may be smooth or crimped or curved.

The heating step may be carried out by means of a straightening iron, a curling iron, a crimping iron or a steam iron such as the Steampod. Preferably, the heating step is carried out using a straightening iron.

As examples of hair irons that may be used in the process according to the invention, mention may be made of any type of flat hair iron, and in particular, in a nonlimiting manner, those described in patents US 5 957 140 and US 5 046 516.

The hair iron may be applied by successive separate strokes lasting a few seconds or by gradual movement or sliding along the locks of keratin fibres, especially of hair.

Preferably, the hair iron is applied in the process according to the invention by a continuous movement from the root to the end of the keratin fibres, especially human keratin fibres such as the hair, in one or more passes, in particular in one to twenty passes. Each pass of the hair iron may last from 2 seconds to 1 minute for a length of the treated keratin fibres of 30 cm ± 3 cm.

Preferably, the step of heating the keratin fibres is performed for a time that may range from 100 milliseconds to 30 minutes, and preferentially from 1 second to 15 minutes, better still from 2 seconds to 10 minutes, better still from 3 seconds to 5 minutes and even better still from 4 seconds to 1 minute.

After the heat treatment step (iii) of the process, the keratin fibres may be optionally rinsed with water or washed with a shampoo. The keratin fibres are subsequently optionally dried using a hairdryer or a hood or naturally.

According to one embodiment, the process according to the invention is performed on keratin fibres, in particular human keratin fibres, such as the hair, especially that are natural, especially natural hair.

According to another embodiment, the process according to the invention is carried out on keratin fibres, especially human keratin fibres, such as the hair, which is damaged. As indicated previously, the term *"damaged hair*" means dry, coarse, brittle, split and/or limp hair.

According to another embodiment, the treatment process according to the invention is preferably carried out on keratin fibres, especially human keratin fibres such as the hair, which are sensitized, such as bleached, artificially dyed, relaxed or permanent-waved fibres.

The process according to the invention may be performed on keratin fibres, especially human keratin fibres such as the hair, which are dry or wet. Preferentially, the process is performed on dry keratin fibres, especially dry hair.

The cosmetic composition(s) used according to the invention are advantageously applied to the keratin fibres in an amount ranging from 0.01 to 10 grams and preferably from 0.1 to 5 grams, more preferably from 0.5 to 3 grams of composition per gram of keratin fibres.

After application of the cosmetic composition to the keratin fibres, they may be wrung out to remove the excess composition.

After application to the keratin fibres of the ingredient a) and/or the ingredient b) as defined above, or of a cosmetic composition containing the same, and before carrying out the keratin fibre heat treatment or heating step (iii), the ingredient(s) a) as defined above and/or the ingredient(s) b) as defined above or the composition(s) containing the same may be left on for a time ranging from 1 to 60 minutes, preferably ranging from 2 to 50 minutes and preferentially ranging from 5 to 45 minutes. The leave-on period may take place at a temperature ranging from 15°C to 45°C, preferably at room temperature (25°C).

Preferably, the process of the invention carries out the steps (i) and (ii) simultaneously, the leave-on period of the ingredients (a) and (b) is then between 1 second and 60 minutes.

According to one embodiment, the process of the invention comprises:
(i) a first step consisting in applying, to said fibres, a) one or more monosaccharides with amine group as defined above, preferably in salified form; then optionally a drying step ii'), then
(ii) a second step consisting in applying, to said fibres, b) one or more polysaccharides with amine group(s) as defined above, preferably that are salified, particularly having an average molecular weight of less than or equal to 400 kDa,
   then optionally a drying step ii'),
   then
(iii) a step of heat treatment at a temperature of at least 80°C, in particular at a temperature between 80°C and 250°C;
it being understood that the ingredient(s) a) as defined above and the ingredient(s) b) as defined above are present in different cosmetic compositions. They are therefore applied separately, i.e. sequentially, to the keratin fibres.

According to another particular variant of the invention, the process of the invention comprises:
(i) a first step consisting in applying, to said fibres, b) one or more polysaccharides with amine group(s) as defined above, preferably in salified form, particularly having an average molecular weight of less than or equal to 400 kDa, then optionally a drying step ii'),
   then
(ii) a second step consisting in applying, to said fibres, a) one or more monosaccharides with amine group as defined above, preferably in salified form; then optionally a drying step ii'),
   then
(iii) a step of heat treatment at a temperature of at least 80°C, in particular at a temperature between 80°C and 250°C;
it being understood that the ingredient(s) a) as defined above and the ingredient(s) b) as defined above are present in different cosmetic compositions. They are therefore applied separately, i.e. sequentially, to the keratin fibres.

According to one particular embodiment, the process of the invention sequentially carries out a first step (i) that consists in applying, to keratin fibres, the monosaccharide(s) with amine group a) as defined above or a composition **(ca)** containing the same, which is/are left on for a time of between 1 min and 30 min inclusive, then a second step (ii) that consists in applying b) the polysaccharide(s) with amine group(s) b) as defined above or a composition (cb) containing same, which is/are left on for between 1 second and 30 minutes, then (ii') said fibres are dried, followed by a heat treatment step (iii).

According to another particular embodiment, the process of the invention carries out a first step (i) that consists in applying, to the keratin fibres, the monosaccharide(s) with amine group a) as defined above simultaneously with the polysaccharide(s) with amine group(s) b) as defined above, which are left on for a time of between 1 min and 60 minutes inclusive, then (ii') said fibres are dried, followed by a heat treatment step (iii).

According to this embodiment, the process of the invention carries out a first step (i) that consists in applying, to the keratin fibres, a composition **(C), (C'), (C"), (C‴),** or **(Cʺʺ)** containing the monosaccharide(s) with amine group a) as defined above and the polysaccharide(s) with amine group(s) b) as defined above, which are left on for a time of between 1 min and 60 min inclusive, then (ii') said fibres are dried, followed by a heat treatment step (iii).

According to a particular embodiment of the process of the invention, the step (i) of applying the monosaccharide(s) with amine group a) uses a composition **(ca)** comprising one or more monosaccharides with amine group a) as defined above in a content ranging between 0.01% and 20% by weight relative to the total weight of the composition which comprises same, more particularly between 0.05% and 10%, more preferentially between 0.1% and 5%, more preferentially still between 0.1% and 1.5% by weight relative to the total weight of the composition comprising same.

According to a particular embodiment of the invention, the step (ii) of the process of applying the polysaccharide(s) with amine group(s) b) uses a composition **(cb)** comprising one or more polysaccharides with amine group(s) b) as defined above in an amount of between 0.01% and 20% by weight relative to the total weight of the composition, more particularly between 0.05% and 10%, more preferentially between 0.1% and 5%, more particularly between 0.1% and 5% by weight relative to the total weight of the composition comprising same.

According to an embodiment of the process according to the invention, the ingredients a) and b) as defined above are present in the same cosmetic composition **(C), (C'), (C"), (C‴),** or **(Cʺʺ).** They are therefore applied simultaneously to the keratin fibres. In this case, the preferred amounts are as follows:
- for the monosaccharide(s) with amine group a) as defined above, the amount is between 0.05% and 15% by weight, relative to the total weight of the composition, preferably between 0.1% and 10% by weight, more preferentially between 0.2% and 6% by weight relative to the total weight of the composition;
- for the polysaccharide(s) with amine group(s) b) as defined above, the specific amount is between 0.01% and 10% by weight, relative to the total weight of the composition, more particularly between 0.1% and 5% by weight relative to the total weight of the composition, preferentially an amount of between 0.01% and 1.5%, more preferentially between 0.1% and 1.1% by weight relative to the total weight of the composition.

The treatment process according to the invention may be carried out before, during and/or after an additional process of cosmetic treatment of the keratin fibres, such as a process for temporarily shaping (shaping with curlers, a crimping iron or a straightening iron) or a process for durably shaping (permanent-waving or relaxing) the keratin fibres.

The treatment process may be performed as a pre-treatment to a dyeing or relaxing process and/or a permanent-waving process so as to cosmetically protect the keratin fibres against these treatments. In other words, this process is performed to preserve the cosmetic properties of the keratin fibres before a dyeing, relaxing and/or a permanent-waving cosmetic treatment process.

The treatment process according to the invention may be performed as a post-treatment to a bleaching, artificial dyeing or relaxing process and/or a permanent-waving process so as to repair the damaged keratin fibres. The keratin fibres may be damaged by overexposure to the environment (for example natural long hair may be photodegraded, or degraded mechanically by repeated brushing or combing).

In particular, the treatment process according to the invention may be performed on damaged keratin fibres.

In other words, the treatment process according to the invention is preferably carried out on sensitized keratin fibres such as bleached, dyed, relaxed or permanent-waved fibres.

In particular, the treatment process may be carried out before a bleaching, dyeing or relaxing process and/or a permanent-waving process on keratin fibres.

### Kit

Another subject of the invention is a multi-compartment kit comprising:
- a first cosmetic composition comprising a) at least one monosaccharide with amine group as defined above and optionally c) one or more organic or mineral acids as defined above, in which the composition has an acid pH, i.e. below 7;
- a second cosmetic composition comprising b) at least one polysaccharide with amine group(s) as defined above and optionally c) one or more organic or mineral acids as defined above, in which the composition has an acid pH, i.e. below 7; and
- a device for heating the keratin fibres at a temperature of at least 80°C, preferably at a temperature of at least 100°C, in particular at a temperature between 100°C and 250°C inclusive. Preferably, the step of heating the keratin fibres is carried out at a temperature ranging from 150°C to 220°C, preferably ranging from 160°C to 220°C, preferentially ranging from 160°C to 200°C, especially ranging from 130°C to 190°C;
the first and second compositions each being packaged in a separate packaging assembly. The contents of each compartment of the kit may be released either sequentially or simultaneously.

The composition packaging assembly is, in a known manner, any packaging that is suitable for storing cosmetic compositions (in particular a bottle, tube, spray bottle or aerosol bottle).

Such a kit allows the process for treating keratin fibres according to the invention to be performed.

The examples that follow are given as illustrations of the present invention.

The amounts indicated in the examples are expressed as weight percentages.

### EXAMPLES

The following compositions were prepared, and the results of the number of fibres is given for each composition.

| **Composition** | **Details of the composition** | **No. of fibres repaired (/5)***** |
|---|---|---|
| **1 comparative** | Glucosamine hydrochloride ("G") at 1% in water, spontaneous pH of 4.8 | 0/5 |
| **2 comparative** | G at 5% in water, spontaneous pH of 4.17 | 0/5 |
| **3 comparative** | Chitosan ("C") at 1% in water* | 0/5 |
| | MW 310-375 K, η = 800-2000 Cp | |
| **4 comparative** | C at 1% in water* | 0/5 |
| | MW approx 5000, η <100 Cp | |
| **5 invention** | GC (G at 1% + C at 1%) in water** with C: MW | 5/5 |
| | 310-375 K, η = 800-2000 Cp | |
| **6 invention** | GC (G at 1% + C at 1%) in water** with C: MW | 5/5 |
| | approx 5000, η <100 Cp | |
| **7 invention** | GC (G at 1% + C at 1%) in water** with C: MW | 5/5 |
| | approx 28 K, η < 100 Cp | |
| **8 invention** | GC (G at 5% + C at 1%) in water** with C: MW | 5/5 |
| | approx 5000, η <100 Cp | |

| | | |
|---|---|---|
| * solubilize by addition of lactic acid up to pH = 4.5 (± 0.5) ** the chitosan composition is adjusted to pH 4.5 (± 0.5) with lactic acid then the glucosamine hydrochloride is added. *** Split-end hair sold by IHIP (Glendale, NY 11385, USA) | | |

The evaluation of the repair of the keratin fibres was carried out using a magnifying optical microscope (Model 175 X optical "MightyScope 1.3M Digital Microscope" sold by Aven Inc, USA - aveninc.com).

Each fibre to be evaluated that is selected for study is a natural fibre and is naturally split into two longitudinally at the end.

The end of each fibre is observed before treatment and after treatment using the microscope. The treatment of the fibres consists in soaking each fibre in the composition for 30 seconds, then each fibre is left suspended for air drying, then is subjected to a heat treatment 3 times successively with a straightening iron at 190°C (Babyliss PRO, EP Technology 5.0 sold by Babyliss).

Each before/after treatment comparison is repeated 5 times on different fibres split at their end longitudinally.

After treatment, it is evaluated using the optical microscope whether the fibre is repaired, i.e. if the two parts of the end of the fibre are joined, the result = 1, if they are not joined, the result = 0.

These results show that the combination of the ingredients a) and b) followed by a heat treatment results in a repair of the fibre, unlike the ingredients a) or b) taken alone.

## Claims

1. Process for treating keratin fibres, especially human keratin fibres, in particular the hair, comprising:
(i) a step consisting in applying, to said fibres, a) one or more monosaccharides with amine group;
(ii) a step consisting in applying, to said fibres, b) one or more polysaccharides with amine group(s);
(ii') optionally a drying step;
(iii) then a step of heat treatment at a temperature above or equal to 80°C, in particular at a temperature between 100°C and 250°C, preferably with a hair iron;
it being understood that the steps (i) and (ii) may be carried out simultaneously, or sequentially, preferably the steps (i) and (ii) are carried out simultaneously and that the drying step (ii'), when it is present, precedes the heat treatment step and follows the steps (i) and (ii);
in which a) the monosaccharide(s) with amine group is (are) in a cosmetic composition having an acid pH, i.e. pH below 7; and
in which b) the polysaccharide(s) with amine group(s) is (are) in a cosmetic composition having an acid pH, i.e. pH below 7; and
in which a) the monosaccharide(s) with amine group is (are) chosen from the compounds of formula **(A')** below and also the organic or mineral acid salts thereof, preferably mineral acid salts thereof, and also the α or β anomers thereof, the optical isomers thereof of L or D configuration, preferably D configuration, and the solvates thereof such as hydrates: 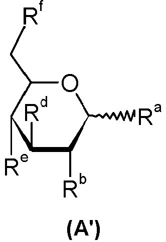
in which formula **(A') R^{b}** represents NH₂, and **R^{a}, R^{d}, R^{e}** and **R^{f}** represent a hydroxyl group; and
in which b) the polysaccharide(s) with amine group(s) is (are) of formula **(B₃)** below, and also the organic or mineral acid salts thereof, and the solvates thereof such as hydrates: 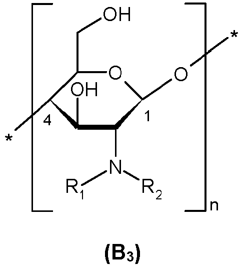
in which formula **(B₃):**
• **R₁** and **R₂** represent i) a hydrogen atom or ii) -C(O)-R'₁ with R'₁ representing a (C₁-C₄)alkyl group ; and
• **n** is an integer greater than or equal to 2, particularly between 3 and 3000 inclusive, more particularly between 5 and 2500, preferentially between 10 and 2300;
it being understood that in the polysaccharide at least one saccharide unit bears an NH₂ amino group and at least one other saccharide unit bears an N(H)-R' group with R' representing a (C₁-C₄)alkylcarbonyl group such as acetyl CH₃-C(O)-.

2. Process according to the preceding claim, in which a) the monosaccharide(s) with amine group is (are) chosen from glucosamine of D configuration and/or salified with a mineral acid such as hydrochloric acid.

3. Process according to any one of the preceding claims, in which a) the monosaccharide(s) with amine group is (are) in a cosmetic composition and is (are) present in said composition in an amount of between 0.01% and 20% by weight relative to the total weight of the composition which comprises same, more particularly between 0.05% and 10%, more preferentially between 0.1% and 5% by weight relative to the total weight of the composition which comprises same.

4. Process according to any one of the preceding claims, in which a) the monosaccharide(s) with amine group is (are) in a cosmetic composition having an acid pH below or equal to 6, more particularly having a pH between 2 and 6 inclusive, preferentially having a pH between 2.5 and 5.5, more preferentially between 3.5 and 5 such as 4.5; particularly the monosaccharide(s) with amine group a) is (are) in the presence of one or more mineral acids, more particularly hydrochloric acid.

5. Process according to any one of the preceding claims, in which b) the polysaccharide(s) with amine group(s) is (are) chosen from chitosans of formula **(B₄)** below, and also the organic or mineral acid salts thereof, and the solvates thereof such as hydrates: 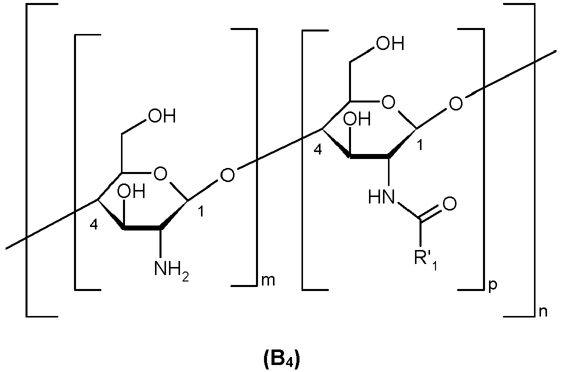 in which formula **(B₄):**
▪ **R'₁** representing a (C₁-C₄)alkyl group such as methyl; and
▪ **p** is greater than 0 and ranges up to 0.5, preferably from 0.05 to 0.3, and better still from 0.1 to 0.20 such as 0.15 with **m+p** being equal to 1;
▪ **n** is an integer greater than or equal to 2, particularly between 3 and 3000 inclusive, more particularly between 5 and 2500, preferentially between 10 and 2300;
it being understood that in the chitosan at least one saccharide unit bears an NH₂ amino group and at least one other saccharide unit bears an N(H)-R', group with R' representing a (C₁-C₄)alkylcarbonyl group such as acetyl CH₃-C(O)-;
particularly the polysaccharide(s) with amine group(s) b) is (are) chosen from chitosans salified using organic acid, preferably using i) monocarboxylic acid of formula **(I) R-C(O)-OH** with **R** representing a (hetero)aryl group such as phenyl, a (hetero)aryl(C₁-C₄)alkyl group such as benzyl, or (C₁-C₃₀)alkyl group or an unsaturated C₂-C₃₀ radical (i.e. comprising at least one ethylenic unsaturation, preferably one ethylenic unsaturation) said alkyl group or unsaturated C₂-C₃₀ radical being optionally interrupted and/or optionally substituted preferably with one or more hydroxyl groups and not substituted with one or more amino radicals, R preferably denoting a (C₁-C₆)alkyl group optionally interrupted and/or optionally substituted with 1, 2 or 3 hydroxyl groups, preferably R represents a (C₁-C₄)alkyl group such as methyl or ethyl; in particular the organic monocarboxylic acids **(I)** are chosen from acetic acid, glycolic acid and lactic acid, more particularly from acetic acid and lactic acid, and xii) the polycarboxylic acids of formula **(II)** below: 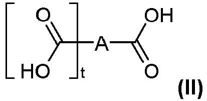
in which formula **(II):**
**A** represents a saturated or unsaturated, cyclic or noncyclic, aromatic or nonaromatic, polyvalent hydrocarbon-based group comprising from 1 to 30 carbon atoms optionally interrupted with one or more heteroatoms such as oxygen and/or optionally substituted in particular with one or more hydroxyl groups and t represents an integer between 1 and 5 inclusive; preferably A represents a divalent (C₁-C₆)alkylene group optionally substituted in particular with one or more hydroxyl groups and not substituted with at least t amino radicals, and t is equal to 1, 2 or 3, the polycarboxylic acids of formula **(II)** are preferably chosen from tartaric acid, succinic acid, fumaric acid and citric acid and more particularly chosen from tartaric acid, succinic acid, fumaric acid, maleic acid and citric acid; and xiii) amino acids comprising more carboxylic acid radicals than amino groups such as gamma-carboxyglutamic acid, aspartic acid, glutamic acid, in particular gamma-carboxyglutamic acid, more preferentially still the polysaccharide(s) with amine group(s) of the invention b) is (are) chosen from chitosans salified using organic monocarboxylic acid of formula **(I)** such as lactate.

6. Process according to any one of the preceding claims, in which b) the polysaccharide(s) with amine group(s) is (are) in a cosmetic composition and is (are) present in said composition which comprises same in an amount of between 0.01% and 20% by weight relative to the total weight of the composition which comprises same, more particularly between 0.05% and 10%, more preferentially between 0.1% and 5% by weight relative to the total weight of the composition which comprises same.

7. Process according to any one of the preceding claims, in which b) the polysaccharide(s) with amine group(s) is (are) in a cosmetic composition having an acid pH below or equal to 6, more particularly having a pH between 2 and 6 inclusive, preferentially having a pH between 2.5 and 5.5, more preferentially between 3.5 and 5 such as 4.5; particularly the polysaccharide(s) with amine group(s) b) is (are) in the presence of one or more organic acids as defined in Claim 5.

8. Process according to any one of the preceding claims, in which b) the polysaccharide(s) with amine group(s) has (have) an average molecular weight MW of less than or equal to 400 kDa, particularly less than 200 kDa, more particularly less than 100 kDa, preferentially less than 40 kDa, more preferentially between 1 kDa and 30 kDa inclusive, more preferentially still have a MW between 3 kDa and 28 kDa inclusive.

9. Process according to any one of the preceding claims, in which steps (i) and (ii) are carried out simultaneously or by applying, to the keratin fibres, a composition comprising one or more monosaccharides with amine group a) as defined in any one of Claims 1 to 4; and one or more polysaccharides with amine group(s) b) as defined in any one of Claims 1, 5 to 8, and optionally one or more organic or mineral acids c).

10. Process according to any one of the preceding claims, in which steps (i) and (ii) are carried out simultaneously or by applying, to the keratin fibres, a composition chosen from:
- the composition **(C')** having an acid pH which comprises:
a) one or more salified monosaccharides with amine group as defined in any one of Claims 1 to 4;
b) one or more salified polysaccharides with amine group(s) as defined in any one of Claims 1, 5 to 8; - the composition **(C")** having an acid pH which comprises:
a) one or more non-salified monosaccharides with amine group as defined in any one of Claims 1 to 4; and
b) one or more salified polysaccharides with amine group(s) as defined in any one of Claims 1, 5 to 8; - the composition **(C‴)** having an acid pH which comprises:
a) one or more salified monosaccharides with amine group as defined in any one of Claims 1 to 4; and
b) one or more non-salified polysaccharides with amine group(s) as defined in any one of Claims 1, 5 to 8; and - the composition **(Cʺʺ)** having an acid pH which comprises:
a) one or more non-salified monosaccharides with amine group as defined in any one of Claims 1 to 4;
b) one or more non-salified polysaccharide(s) with amine group(s) as defined in any one of Claims 1, 5 to 8; and
c) one or more organic or mineral acids.

11. Process according to any one of Claims 1 to 8, which comprises:
(i) a first step consisting in applying, to said fibres, a) one or more monosaccharides with amine group according to any one of Claims 1 to 4; then
(ii) a second step consisting in applying, to said fibres, b) one or more polysaccharides with amine group(s) as defined in any one of Claims 1, 5 to 8; then
(iii) a step of heat treatment above or equal to 80°C, in particular at a temperature between 100°C and 250°C, preferably with a hair iron;
it being understood that the ingredient(s) a) as defined above and the ingredient(s) b) as defined above are present in different cosmetic compositions.

12. Process according to any one of the preceding claims, in which the step of heat treatment or step of heating said fibres (iii) is carried out at a temperature of at least 100°C, in particular at a temperature between 100°C and 250°C inclusive; preferably, the step of heating the keratin fibres is carried out at a temperature ranging from 150°C to 220°C, preferably ranging from 160°C to 220°C, preferentially ranging from 160°C to 200°C, especially ranging from 130°C to 190°C.

13. Process according to any one of the preceding claims, which does not use oxidized polysaccharide; more generally which does not use a compound comprising at least one aldehyde group.

14. Process according to any one of the preceding claims, in which a step (ii') of drying the keratin fibres, in particular human fibres such as the hair, is carried out before the heat treatment step (iii), said drying is in particular carried out using a hairdryer, a hood, or else with a towel, an absorbent or by natural drying, the drying step is advantageously carried out at a temperature ranging from 20°C to 70°C.

15. Acid composition **(C)** i.e. which has a pH below 7, in particular below or equal to 6, more particularly having a pH between 2 and 6 inclusive, preferentially having a pH between 2.5 and 5.5, more preferentially between 3.5 and 5 such as 4.5; and which comprises:
either
a) one or more salified monosaccharides with amine group as defined in any one of Claims 1 to 4;
b) one or more salified polysaccharides with amine group(s) as defined in any one of Claims 1, 5 to 8;
and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid and alginic acid;
or
a) one or more non-salified monosaccharides with amine group as defined in any one of Claims 1 to 4;
b) one or more salified polysaccharides with amine group(s) as defined in any one of Claims 1, 5 to 8;
and optionally c) one or more mineral or organic acids preferably different from the following acids: aspartic acid, glutamic acid, gluconic acid, pyrrolidonecarboxylic acid, 100 OE and 500 OE ethoxylated stearic acid, linoleic acid, succinic acid and alginic acid;
or
a) one or more salified monosaccharides with amine group as defined in any one of Claims 1 to 4;
b) one or more non-salified polysaccharides with amine group(s) as defined in any one of Claims 1, 5 to 8;
and c) one or more mineral or organic acids different from the following acids: aspartic acid, glutamic acid, gluconic acid, pyrrolidonecarboxylic acid, 100 OE and 500 OE ethoxylated stearic acid, linoleic acid, succinic acid and alginic acid ;
or
a) one or more non-salified monosaccharides with amine group as defined in any one of Claims 1 to 4;
b) one or more non-salified polysaccharides with amine group(s) as defined in any one of Claims 1, 5 to 8; and
c) one or more mineral or organic acids different from the following acids: aspartic acid, glutamic acid, gluconic acid, pyrrolidonecarboxylic acid, 100 OE and 500 OE ethoxylated stearic acid, linoleic acid, and succinic acid and alginic acid.

16. Composition as defined previously which does not contain oxidized polysaccharide; more particularly the composition does not comprise a compound comprising at least one aldehyde group.

17. Use a) of one or more monosaccharides with amine group as defined in any one of Claims 1 to 4 in which a) the monosaccharide(s) with amine group is (are) in a cosmetic composition having an acid pH, i.e. pH below 7; and b) of one or more polysaccharides with amine group(s) as defined in any one of Claims 1, 5 to 8 in which b) the polysaccharide(s) with amine group(s) is (are) in a cosmetic composition having an acid pH, i.e. pH below 7, to improve the cosmetic effect of the keratin fibres intrinsically, to prevent or reduce the breakage of natural keratin fibres and/or to repair damaged keratin fibres, while retaining or even improving the manageability, anti-frizz properties and/or persistence of the styling of said fibres, in particular human fibres such as the hair, when a fibre heating step (iii) is carried out at at least 80°C, in particular at a temperature between 100°C and 250°C, preferably with a hair iron.

18. Multi-compartment kit comprising:
• a first cosmetic composition comprising a) one or more monosaccharides with amine group as defined in any one of Claims 1 to 4, and optionally c) one or more organic or mineral acids, in which the composition has an acid pH, i.e. below 7;
• a second cosmetic composition comprising b) one or more polysaccharides with amine group(s) as defined in any one of Claims 1, 5 to 8, and optionally c) one or more organic or mineral acids, in which the composition has an acid pH, i.e. below 7; and
• a device for heating the keratin fibres at a temperature of at least 80°C, preferably at a temperature of at least 100°C, in particular at a temperature between 100°C and 250°C inclusive; preferably, the step of heating the keratin fibres is carried out at a temperature ranging from 150°C to 220°C, preferably ranging from 160°C to 220°C, preferentially ranging from 160°C to 200°C, especially ranging from 130°C to 190°C;
the first and second compositions each being packaged in a separate packaging assembly.

## Patentansprüche

1. Verfahren zur Behandlung von Keratinfasern, insbesondere menschlichen Keratinfasern, insbesondere dem Haar, umfassend:
(i) einen Schritt, der darin besteht, dass man auf die Fasern a) ein oder mehrere Monosaccharide mit Amingruppe aufbringt;
(ii) einen Schritt, der darin besteht, dass man auf die Fasern b) ein oder mehrere Polysaccharide mit Amingruppe(n) aufbringt;
(ii') gegebenenfalls einen Trocknungsschritt;
(iii) dann einen Schritt der Wärmebehandlung bei einer Temperatur über oder gleich 80 °C, insbesondere bei einer Temperatur zwischen 100 °C und 250 °C, vorzugsweise mit einem Haareisen;
mit der Maßgabe, dass die Schritte (i) und (ii) gleichzeitig oder nacheinander durchgeführt werden können und die Schritte (i) und (ii) vorzugsweise gleichzeitig durchgeführt werden und dass der Trocknungsschritt (ii'), wenn er vorhanden ist, dem Wärmebehandlungsschritt vorgeschaltet ist und auf die Schritte (i) und (ii) folgt;
wobei a) das Monosaccharid bzw. die Monosaccharide mit Amingruppe in einer kosmetischen Zusammensetzung mit einem sauren pH-Wert, d. h. einem pH-Wert unter 7, vorliegt bzw. vorliegen; und
wobei b) das Polysaccharid bzw. die Polysaccharide mit Amingruppe(n) in einer kosmetischen Zusammensetzung mit einem sauren pH-Wert, d. h. einem pH-Wert unter 7, vorliegt bzw. vorliegen; und
wobei a) das Monosaccharid bzw. die Monosaccharide mit Amingruppe aus den Verbindungen der nachstehenden Formel (A') sowie den Salzen davon mit einer organischen oder mineralischen Säure, vorzugsweise Salzen davon mit einer mineralischen Säure, sowie den α- oder β-Anomeren davon, den optischen Isomeren davon mit L- oder D-Konfiguration, vorzugsweise D-Konfiguration, und den Solvaten davon wie Hydraten ausgewählt ist bzw. sind: 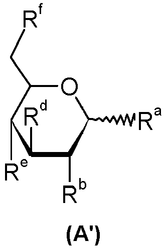
wobei in der Formel (A') R^{b} für NH₂ steht und R^{a}, R^{d}, R^{e} und R^{f} für eine Hydroxylgruppe stehen; und
wobei b) das Polysaccharid bzw. die Polysaccharide mit Amingruppe(n) die nachstehende Formel (B₃) aufweisen, sowie die Salze davon mit einer organischen oder mineralischen Säure und die Solvate davon wie Hydrate: 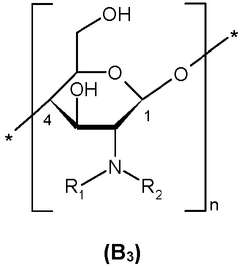
wobei in der Formel (B₃):
• R₁ und R₂ für i) ein Wasserstoffatom oder ii) -C(O)-R'₁ stehen, wobei R'₁ für eine (C₁-C₄)Alkylgruppe steht; und
• n eine ganze Zahl größer oder gleich 2, insbesondere zwischen 3 und 3000 inklusive, spezieller zwischen 5 und 2500, bevorzugt zwischen 10 und 2300, ist;
mit der Maßgabe, dass in dem Polysaccharid mindestens eine Saccharideinheit eine NH₂ -Aminogruppe trägt und mindestens eine andere Saccharideinheit eine N(H)-R'-Gruppe trägt, wobei R' für eine (C₁-C₄)Alkylcarbonylgruppe wie Acetyl CH₃-C(O)- steht.

2. Verfahren nach dem vorhergehenden Anspruch, wobei a) das Monosaccharid bzw. die Monosaccharide mit Amingruppe aus Glucosamin, das D-Konfiguration aufweist und/oder mit einer mineralischen Säure wie Salzsäure versalzt ist, ausgewählt ist bzw. sind.

3. Verfahren nach einem der vorhergehenden Ansprüche, wobei a) das Monosaccharid bzw. die Monosaccharide mit Amingruppe in einer kosmetischen Zusammensetzung vorliegt bzw. vorliegen und in der Zusammensetzung in einer Menge zwischen 0,01 und 20 Gew.-%, bezogen auf das Gesamtgewicht der Zusammensetzung, die es bzw. sie umfasst, spezieller zwischen 0,05 und 10 Gew.-%, weiter bevorzugt zwischen 0,1 und 5 Gew.-%, bezogen auf das Gesamtgewicht der Zusammensetzung, die es bzw. sie umfasst, vorliegt bzw. vorliegen.

4. Verfahren nach einem der vorhergehenden Ansprüche, wobei a) das Monosaccharid bzw. die Monosaccharide mit Amingruppe in einer kosmetischen Zusammensetzung mit einem sauren pH-Wert unter oder gleich 6, spezieller mit einem pH-Wert zwischen 2 und 6 inklusive, vorzugsweise mit einem pH-Wert zwischen 2,5 und 5,5, weiter bevorzugt zwischen 3,5 und 5, wie 4,5, vorliegt bzw. vorliegen; insbesondere das Monosaccharid bzw. die Monosaccharide mit Amingruppe a) in Gegenwart einer oder mehrerer mineralischer Säuren, spezieller Salzsäure, vorliegt bzw. vorliegen.

5. Verfahren nach einem der vorhergehenden Ansprüche, wobei b) das Polysaccharid bzw. die Polysaccharide mit Amingruppe(n) aus Chitosanen der folgenden Formel (B₄) sowie Salzen davon mit einer organischen oder mineralischen Säure und den Solvaten davon wie Hydraten ausgewählt ist bzw. sind: 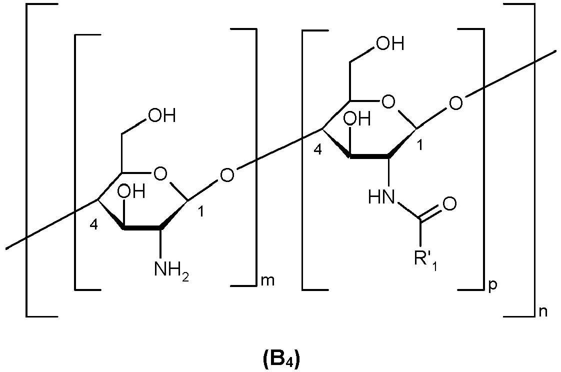 wobei in der Formel (B₄):
▪ R'₁ für eine (C₁-C₄)-Alkylgruppe wie Methyl steht; und
▪ p größer als 0 ist und im Bereich bis zu 0,5, vorzugsweise von 0,05 bis 0,3 und noch besser von 0,1 bis 0,20, wie 0,15, liegt, wobei m+p gleich 1 ist;
▪ n eine ganze Zahl größer oder gleich 2, insbesondere zwischen 3 und 3000 inklusive, spezieller zwischen 5 und 2500, bevorzugt zwischen 10 und 2300, ist;
mit der Maßgabe, dass in dem Chitosan mindestens eine Saccharideinheit eine NH₂ -Aminogruppe trägt und mindestens eine andere Saccharideinheit eine N(H)-R'₁-Gruppe trägt, wobei R' für eine (C₁-C₄)Alkylcarbonylgruppe wie Acetyl CH₃-C(O)- steht;
insbesondere das Polysaccharid bzw. die Polysaccharide mit Amingruppe(n) b) aus Chitosanen ausgewählt ist bzw. sind, die mit einer organischen Säure versalzt sind, vorzugsweise unter Verwendung von i) Monocarbonsäure der Formel (I) R-C(O)-OH, wobei R für eine (Hetero)arylgruppe wie Phenyl, eine (Hetero)aryl-(C₁-C₄)-alkylgruppe wie Benzyl oder (C₁-C₃₀)-Alkylgruppe oder einen ungesättigten C₂-C₃₀-Rest (d. h. mit mindestens einer ethylenischen Ungesättigtheit, vorzugsweise einer ethylenischen Ungesättigtheit) steht, wobei die Alkylgruppe oder der ungesättigte C₂-C₃₀-Rest gegebenenfalls unterbrochen und/oder gegebenenfalls vorzugsweise durch eine oder mehrere Hydroxylgruppen substituiert ist und nicht durch eine oder mehreren Aminoreste substituiert ist, wobei R vorzugsweise eine (C₁-C₆)-Alkylgruppe, die gegebenenfalls unterbrochen und/oder gegebenenfalls durch 1, 2 oder 3 Hydroxylgruppen substituiert ist, bedeutet, R vorzugsweise für eine (C₁-C₄)-Alkylgruppe wie Methyl oder Ethyl steht; insbesondere die organischen Monocarbonsäuren (I) aus Essigsäure, Glykolsäure und Milchsäure, spezieller aus Essigsäure und Milchsäure ausgewählt sind, und xii) den Polycarbonsäuren der folgenden Formel (II): 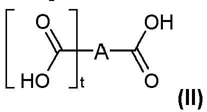
wobei in Formel (II):
A für eine gesättigte oder ungesättigte, cyclische oder nichtcyclische, aromatische oder nichtaromatische, mehrwertige Gruppe auf Kohlenwasserstoffbasis mit 1 bis 30 Kohlenstoffatomen, die gegebenenfalls durch ein oder mehrere Heteroatome wie Sauerstoff unterbrochen ist und/oder gegebenenfalls insbesondere durch eine oder mehrere Hydroxylgruppen substituiert ist, steht und t für eine ganze Zahl zwischen 1 und 5 inklusive steht; vorzugsweise A für eine zweiwertige (C₁-C₆)-Alkylengruppe, die gegebenenfalls insbesondere durch eine oder mehrere Hydroxylgruppen substituiert ist und nicht durch mindestens t Aminoreste substituiert ist, steht und t gleich 1, 2 oder 3 ist, die Polycarbonsäuren der Formel (II) vorzugsweise aus Weinsäure, Bernsteinsäure, Fumarsäure und Citronensäure ausgewählt sind und spezieller aus Weinsäure, Bernsteinsäure, Fumarsäure, Maleinsäure und Citronensäure ausgewählt sind; und xiii) Aminosäuren mit mehr Carbonsäureresten als Aminogruppen wie gamma-Carboxyglutaminsäure, Asparaginsäure, Glutaminsäure, insbesondere gamma-Carboxyglutaminsäure, noch weiter bevorzugt das Polysaccharid bzw. die Polysaccharide mit Amingruppe(n) der Erfindung b) aus Chitosanen, die mit organischer Monocarbonsäure der Formel (I) wie Lactat versalzt sind, ausgewählt ist bzw. sind.

6. Verfahren nach einem der vorhergehenden Ansprüche, wobei b) das Polysaccharid bzw. die Polysaccharide mit Amingruppe(n) in einer kosmetischen Zusammensetzung vorliegt bzw. vorliegen und in der Zusammensetzung in einer Menge zwischen 0,01 und 20 Gew.-%, bezogen auf das Gesamtgewicht der Zusammensetzung, die es bzw. sie umfasst, spezieller zwischen 0,05 und 10 Gew.-%, weiter bevorzugt zwischen 0,1 und 5 Gew.-%, bezogen auf das Gesamtgewicht der Zusammensetzung, die es bzw. sie umfasst, vorliegt bzw. vorliegen.

7. Verfahren nach einem der vorhergehenden Ansprüche, wobei b) das Polysaccharid bzw. die Polysaccharide mit Amingruppe(n) in einer kosmetischen Zusammensetzung mit einem sauren pH-Wert unter oder gleich 6, spezieller mit einem pH-Wert zwischen 2 und 6 inklusive, vorzugsweise mit einem pH-Wert zwischen 2,5 und 5,5, weiter bevorzugt zwischen 3,5 und 5, wie 4,5, vorliegt bzw. vorliegen; insbesondere das Polysaccharid bzw. die Polysaccharide mit Amingruppe(n) b) in Gegenwart einer oder mehrerer organischer Säuren gemäß Anspruch 5 vorliegt bzw. vorliegen.

8. Verfahren nach einem der vorhergehenden Ansprüche, wobei b) das Polysaccharid bzw. die Polysaccharide mit Amingruppe(n) ein mittleres Molekulargewicht MW von weniger als oder gleich 400 kDa, insbesondere weniger als 200 kDa, spezieller weniger als 100 kDa, bevorzugt weniger als 40 kDa, weiter bevorzugt zwischen 1 kDa und 30 kDa inklusive, noch weiter bevorzugt ein MW zwischen 3 kDa und 28 kDa inklusive aufweisen.

9. Verfahren nach einem der vorhergehenden Ansprüche, wobei die Schritte (i) und (ii) gleichzeitig oder durch Aufbringen einer Zusammensetzung, die ein oder mehrere Monosaccharide mit Amingruppe a) gemäß einem der Ansprüche 1 bis 4 und ein oder mehrere Polysaccharide mit Amingruppe(n) b) gemäß einem der Ansprüche 1, 5 bis 8 und gegebenenfalls eine oder mehrere organische oder mineralische Säuren c) umfasst, auf die Keratinfasern durchgeführt werden.

10. Verfahren nach einem der vorhergehenden Ansprüche, wobei die Schritte (i) und (ii) gleichzeitig oder durch Aufbringen einer Zusammensetzung auf die Keratinfasern durchgeführt werden, wobei die Zusammensetzung ausgewählt ist aus:
- der Zusammensetzung (C') mit einem sauren pH-Wert, die Folgendes umfasst:
a) ein oder mehrere versalzte Monosaccharide mit Amingruppe gemäß einem der Ansprüche 1 bis 4;
b) ein oder mehrere versalzte Polysaccharide mit Amingruppe(n) gemäß einem der Ansprüche 1, 5 bis 8; - der Zusammensetzung (C") mit einem sauren pH-Wert, die Folgendes umfasst:
a) ein oder mehrere nicht versalzte Monosaccharide mit Amingruppe gemäß einem der Ansprüche 1 bis 4; und
b) ein oder mehrere versalzte Polysaccharide mit Amingruppe(n) gemäß einem der Ansprüche 1, 5 bis 8; - der Zusammensetzung (C‴) mit einem sauren pH-Wert, die Folgendes umfasst:
a) ein oder mehrere versalzte Monosaccharide mit Amingruppe gemäß einem der Ansprüche 1 bis 4; und
b) ein oder mehrere nicht versalzte Polysaccharide mit Amingruppe(n) gemäß einem der Ansprüche 1, 5 bis 8; und
- der Zusammensetzung (C"") mit einem sauren pH-Wert, die Folgendes umfasst:
a) ein oder mehrere nicht versalzte Monosaccharide mit Amingruppe gemäß einem der Ansprüche 1 bis 4;
b) ein oder mehrere nicht versalzte Polysaccharide mit Amingruppe(n) gemäß einem der Ansprüche 1, 5 bis 8; und
c) eine oder mehrere organische oder mineralische Säuren.

11. Verfahren nach einem der Ansprüche 1 bis 8, das Folgendes umfasst:
(i) einen ersten Schritt, der darin besteht, dass man auf die Fasern a) ein oder mehrere Monosaccharide mit Amingruppe gemäß einem der Ansprüche 1 bis 4 aufbringt; dann
(ii) einen zweiten Schritt, der darin besteht, dass man auf die Fasern b) ein oder mehrere Polysaccharide mit Amingruppe(n) gemäß einem der Ansprüche 1, 5 bis 8 aufbringt; dann
(iii) einen Schritt der Wärmebehandlung über oder gleich 80 °C, insbesondere bei einer Temperatur zwischen 100 °C und 250 °C, vorzugsweise mit einem Haareisen;
mit der Maßgabe, dass der Inhaltsstoff bzw. die Inhaltsstoffe a) gemäß obiger Definition und der Inhaltsstoff bzw. die Inhaltsstoffe b) gemäß obiger Definition in verschiedenen kosmetischen Zusammensetzungen vorliegen.

12. Verfahren nach einem der vorhergehenden Ansprüche, wobei der Schritt der Wärmebehandlung oder der Schritt des Erhitzens der Fasern (iii) bei einer Temperatur von mindestens 100 °C, insbesondere bei einer Temperatur zwischen 100 °C und 250 °C inklusive, durchgeführt wird; vorzugsweise der Schritt des Erhitzens der Keratinfasern bei einer Temperatur im Bereich von 150 °C bis 220 °C, vorzugsweise im Bereich von 160 °C bis 220 °C, bevorzugt im Bereich von 160 °C bis 200 °C, insbesondere im Bereich von 130 °C bis 190 °C, durchgeführt wird.

13. Verfahren nach einem der vorhergehenden Ansprüche, wobei kein oxidiertes Polysaccharid verwendet wird; allgemeiner bei dem keine Verbindung mit mindestens einer Aldehydgruppe verwendet wird.

14. Verfahren nach einem der vorhergehenden Ansprüche, wobei ein Schritt (ii') des Trocknens der Keratinfasern, insbesondere menschlicher Fasern wie des Haars, vor dem Wärmebehandlungsschritt (iii) durchgeführt wird, wobei das Trocknen insbesondere unter Verwendung eines Haartrockners, einer Haube oder auch mit einem Handtuch, einem Absorptionsmittel oder durch natürliches Trocknen durchgeführt wird und der Trocknungsschritt vorteilhafterweise bei einer Temperatur im Bereich von 20 °C bis 70 °C durchgeführt wird.

15. Saure Zusammensetzung (C), d. h. mit einem pH-Wert unter 7, insbesondere unter oder gleich 6, spezieller mit einem pH-Wert zwischen 2 und 6 inklusive, bevorzugt mit einem pH-Wert zwischen 2,5 und 5,5, weiter bevorzugt zwischen 3,5 und 5, wie 4,5; die Folgendes umfasst:
entweder
a) ein oder mehrere versalzte Monosaccharide mit Amingruppe gemäß einem der Ansprüche 1 bis 4;
b) ein oder mehrere Polysaccharide mit Amingruppe(n) gemäß einem der Ansprüche 1, 5 bis 8;
und gegebenenfalls c) eine oder mehrere mineralische oder organische Säuren, die vorzugsweise von den folgenden Säuren verschieden sind: Asparaginsäure, Glutaminsäure, Gluconsäure, Pyrrolidoncarbonsäure, ethoxylierte 100-OE- und 500-OE-Stearinsäure, Linolsäure, Bernsteinsäure und Alginsäure;
oder
a) ein oder mehrere nicht versalzte Monosaccharide mit Amingruppe gemäß einem der Ansprüche 1 bis 4;
b) ein oder mehrere Polysaccharide mit Amingruppe(n) gemäß einem der Ansprüche 1, 5 bis 8;
und gegebenenfalls c) eine oder mehrere mineralische oder organische Säuren, die vorzugsweise von den folgenden Säuren verschieden sind: Asparaginsäure, Glutaminsäure, Gluconsäure, Pyrrolidoncarbonsäure, ethoxylierte 100-OE- und 500-OE-Stearinsäure, Linolsäure, Bernsteinsäure und Alginsäure;
oder
a) ein oder mehrere versalzte Monosaccharide mit Amingruppe gemäß einem der Ansprüche 1 bis 4;
b) ein oder mehrere nicht versalzte Polysaccharide mit Amingruppe(n) gemäß einem der Ansprüche 1, 5 bis 8;
und c) eine oder mehrere mineralische oder organische Säuren, die von den folgenden Säuren verschieden sind: Asparaginsäure, Glutaminsäure, Gluconsäure, Pyrrolidoncarbonsäure, ethoxylierte 100-OE- und 500-OE-Stearinsäure, Linolsäure, Bernsteinsäure und Alginsäure;
oder
a) ein oder mehrere nicht versalzte Monosaccharide mit Amingruppe gemäß einem der Ansprüche 1 bis 4;
b) ein oder mehrere nicht versalzte Polysaccharide mit Amingruppe(n) gemäß einem der Ansprüche 1, 5 bis 8; und
c) eine oder mehrere mineralische oder organische Säuren, die von den folgenden Säuren verschieden sind: Asparaginsäure, Glutaminsäure, Gluconsäure, Pyrrolidoncarbonsäure, ethoxylierte 100-OE- und 500-OE-Stearinsäure, Linolsäure, Bernsteinsäure und Alginsäure;

16. Zusammensetzung gemäß obiger Definition, die kein oxidiertes Polysaccharid enthält; wobei die Zusammensetzung spezieller keine Verbindung mit mindestens einer Aldehydgruppe umfasst.

17. Verwendung a) eines oder mehrerer Monosaccharide mit Amingruppe gemäß einem der Ansprüche 1 bis 4, wobei a) das Monosaccharid bzw. die Monosaccharide mit Amingruppe in einer kosmetischen Zusammensetzung mit einem sauren pH-Wert, d. h. einem pH-Wert unter 7, vorliegt bzw. vorliegen; und b) eines oder mehrerer Polysaccharide mit Amingruppe(n) gemäß einem der Ansprüche 1, 5 bis 8, wobei b) das Polysaccharid bzw. die Polysaccharide mit Amingruppe(n) in einer kosmetischen Zusammensetzung mit einem sauren pH-Wert, d. h. einem pH-Wert unter 7, vorliegt bzw. vorliegen, zur intrinsischen Verbesserung der kosmetischen Wirkung der Keratinfasern, zur Verhinderung oder Verringerung des Bruchs natürlicher Keratinfasern und/oder zur Reparatur geschädigter Keratinfasern unter Beibehaltung oder sogar Verbesserung der Handhabbarkeit, der Anti-Frizz-Eigenschaften und/oder der Persistenz des Stylings der Fasern, insbesondere menschlicher Fasern wie dem Haar, bei Durchführung eines Fasererhitzungsschritts (iii) bei mindestens 80 °C, insbesondere bei einer Temperatur zwischen 100 °C und 250 °C, vorzugsweise mit einem Haareisen.

18. Kit mit mehreren Kompartimenten, umfassend
• eine erste kosmetische Zusammensetzung, umfassend a) ein oder mehrere Monosaccharide mit Amingruppe gemäß einem der Ansprüche 1 bis 4 und gegebenenfalls c) eine oder mehrere organische oder mineralische Säuren, wobei die Zusammensetzung einen sauren pH-Wert, d. h. unter 7, aufweist;
• eine zweite kosmetische Zusammensetzung, umfassend b) ein oder mehrere Polysaccharide mit Amingruppe(n) gemäß einem der Ansprüche 1, 5 bis 8 und gegebenenfalls c) eine oder mehrere organische oder mineralische Säuren, wobei die Zusammensetzung einen sauren pH-Wert, d. h. unter 7, aufweist; und
• eine Vorrichtung zum Erhitzen der Keratinfasern auf eine Temperatur von mindestens 80 °C, vorzugsweise auf eine Temperatur von mindestens 100 °C, insbesondere auf eine Temperatur zwischen 100 °C und 250 °C inklusive; wobei der Schritt des Erhitzens der Keratinfasern vorzugsweise bei einer Temperatur im Bereich von 150 °C bis 220 °C, vorzugsweise im Bereich von 160 °C bis 220 °C, bevorzugt im Bereich von 160 °C bis 200 °C, insbesondere im Bereich von 130 °C bis 190 °C, durchgeführt wird;
wobei die erste Zusammensetzung und die zweite Zusammensetzung jeweils in einer separaten Verpackungsanordnung verpackt sind.

## Revendications

1. Procédé de traitement de fibres kératiniques, notamment de fibres kératiniques humaines, en particulier des cheveux, comprenant :
(i) une étape d'application, sur lesdites fibres, a) d'un ou plusieurs monosaccharides comportant un groupe amine ;
(ii) une étape d'application, sur lesdites fibres, b) d'un ou plusieurs polysaccharides comportant un ou plusieurs groupes amine ;
(ii') éventuellement une étape de séchage ;
(iii) puis une étape de traitement thermique à une température supérieure ou égale à 80 °C, en particulier à une température comprise entre 100 °C et 250 °C, de préférence avec un fer à cheveux ;
étant entendu que les étapes (i) et (ii) peuvent être réalisées simultanément ou de manière séquentielle, de préférence les étapes (i) et (ii) sont réalisées simultanément et l'étape de séchage (ii'), lorsqu'elle est présente, précède l'étape de traitement thermique et suit les étapes (i) et (ii) ;
dans lequel a) le ou les monosaccharides comportant un groupe amine sont dans une composition cosmétique ayant un pH acide, c'est-à-dire pH inférieur à 7 ; et
dans lequel b) le ou les polysaccharides comportant un ou plusieurs groupes amine sont dans une composition cosmétique ayant un pH acide, c'est-à-dire pH inférieur à 7 ; et
dans lequel a) le ou les monosaccharides comportant un groupe amine sont choisis parmi les composés de formule (A') ci-dessous et également leurs sels d'acide organique ou minéral, de préférence minéral, et également leurs anomères α ou β, leurs isomères optiques de configuration L ou D, de préférence de configuration D et leurs solvates tels que les hydrates : 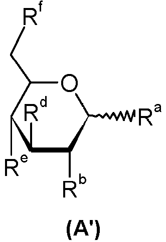
Formule (A') dans laquelle R^{b} représente NH₂, R^{a}, R^{d}, R^{e} et R^{f} représentent un groupe hydroxy ; et
dans lequel b) le ou les polysaccharides comportant un ou plusieurs groupes amine sont de formule (B₃) ci-dessous et également leurs sels d'acide organique ou minéral, et leurs solvates tels que les hydrates, 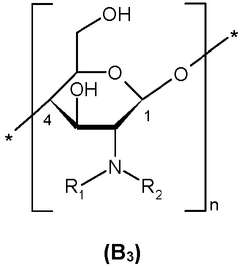
formule (B₃) dans laquelle :
• R₁ et R₂ représentent i) un atome d'hydrogène ou ii) -C(O)-R'₁, R'₁ représentant un groupe (C₁-C₄)alkyle ; et
• n est un entier supérieur ou égal à 2, particulièrement compris inclusivement entre 3 et 3 000, plus particulièrement entre 5 et 2 500, préférentiellement entre 10 et 2 300 ;
étant entendu que dans le polysaccharide au moins une unité saccharide porte un groupe amino NH₂ et au moins une autre unité saccharide porte un groupe N(H)-R', R' représentant un groupe (C₁-C₄)alkylcarbonyle tel qu'acétyle CH₃-C(O)-.

2. Procédé selon la revendication précédente, dans lequel a) le ou les monosaccharides comportant un groupe amine sont choisis parmi la glucosamine de configuration D et/ou salifiés par un acide minéral tel que l'acide chlorhydrique.

3. Procédé selon une quelconque des revendications précédentes dans lequel a) le ou les monosaccharides comportant un groupe amine sont dans une composition cosmétique et sont présents dans ladite composition en une quantité comprise entre 0,01 % et 20 % en poids par rapport au poids total de la composition qui les comprend, plus particulièrement entre 0,05 % et 10 %, plus préférentiellement entre 0,1 % et 5 % en poids par rapport au poids total de la composition qui les comprend.

4. Procédé selon une quelconque des revendications précédentes, dans lequel a) le ou les monosaccharides comportant un groupe amine sont dans une composition cosmétique ayant un pH acide inférieur ou égal à 6, plus particulièrement ayant un pH compris inclusivement entre 2 et 6, préférentiellement ayant un pH compris entre 2,5 et 5,5, plus préférentiellement entre 3,5 et 5, tel que 4,5 ; particulièrement le ou les monosaccharides comportant un groupe amine a) sont en la présence d'un ou plusieurs acides minéraux, plus particulièrement l'acide chlorhydrique.

5. Procédé selon une quelconque des revendications précédentes, dans lequel b) le ou les polysaccharides comportant un ou plusieurs groupes amine sont choisis parmi les chitosanes de formule (B₄) ci-dessous et également leurs sels d'acide organique ou minéral, et leurs solvates tels que les hydrates : 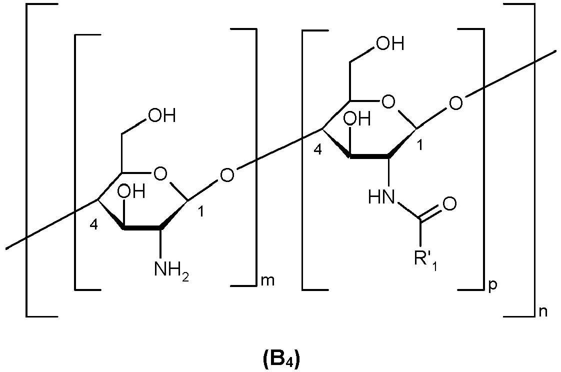 formule (B₄) dans laquelle :
▪ R'₁ représente un groupe (C₁-C₄)alkyle tel que méthyle ; et
▪ p est supérieur à 0 et va jusqu'à 0,5, de préférence de 0,05 à 0,3, et mieux de 0,1 à 0,20 tel que 0,15, m+p étant égal à 1 ;
▪ n est un entier supérieur ou égal à 2, particulièrement compris inclusivement entre 3 et 3 000, plus particulièrement entre 5 et 2 500, préférentiellement entre 10 et 2 300 ;
étant entendu que dans le chitosane au moins une unité saccharide porte un groupe amino NH₂ et au moins une autre unité saccharide porte un groupe N(H)-R'₁, R' représentant un groupe (C₁-C₄)alkylcarbonyle tel qu'acétyle CH₃-C(O)- ;
particulièrement le ou les polysaccharides comportant un ou plusieurs groupes amine b) sont choisis parmi les chitosanes salifiés en utilisant un acide organique, de préférence en utilisant un i) acide monocarboxylique de formule (I) R-C(O)-OH, R représentant un groupe (hétéro)aryle tel que phényle, (hétéro)aryl(C₁-C₄)alkyle tel que benzyle, ou un radical (C₁-C₃₀)alkyle ou un radical insaturé en C₂-C₃₀ (c'est-à-dire comprenant au moins une insaturation éthylénique, de préférence une insaturation éthylénique), ledit groupe alkyle ou radical insaturé en C₂-C₃₀ étant éventuellement interrompu et/ou éventuellement substitué de préférence par un ou plusieurs groupes hydroxyle et non substitué par un ou plusieurs radicaux amino, de préférence R désignant un groupe (C₁-C₆)alkyle éventuellement interrompu et/ou éventuellement substitué par 1, 2 ou 3 groupes hydroxy, de préférence R représente un groupe (C₁-C₄)alkyle tel que méthyle ou éthyle ; en particulier les acides monocarboxyliques organiques (I) sont choisis parmi l'acide acétique, l'acide glycolique et l'acide lactique, plus particulièrement parmi l'acide acétique et l'acide lactique, et xii) les acides polycarboxyliques de formule (II) ci-dessous : 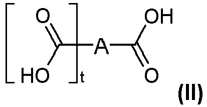
dans laquelle formule (II) :
A représente un groupe à base d'hydrocarbure polyvalent, saturé ou insaturé, cyclique ou non cyclique, aromatique ou non aromatique comprenant de 1 à 30 atomes de carbone éventuellement interrompu par un ou plusieurs hétéroatomes tels qu'oxygène et/ou éventuellement substitué en particulier par un ou plusieurs groupes hydroxyle et t représente un entier compris inclusivement entre 1 et 5 ; de préférence A représente un groupe divalent (C₁-C₆)alkylène éventuellement substitué en particulier par un ou plusieurs groupes hydroxyle et non substitué par au moins t radicaux amino, et t est égal à 1, 2 ou 3, les polyacides carboxyliques de formule (II) sont de préférence choisis parmi l'acide tartrique, l'acide succinique, l'acide fumarique, l'acide citrique, et plus particulièrement choisis parmi l'acide tartrique, l'acide succinique, l'acide fumarique, l'acide maléique et l'acide citrique ; et xiii) les acides aminés comprenant plus de radicaux acide carboxylique que de groupes amino tels que l'acide gamma-carboxyglutamique, l'acide aspartique, l'acide glutamique, en particulier l'acide gamma-carboxyglutamique; plus préférentiellement encore le ou les polysaccharides comportant un ou plusieurs groupes amine de l'invention b) sont choisis parmi des chitosanes salifiés en utilisant un acide monocarboxylique organique de formule (I) tel que lactate.

6. Procédé selon une quelconque des revendications précédentes, dans lequel b) le ou les polysaccharides comportant un ou plusieurs groupes amine sont dans une composition cosmétique et sont présents dans ladite composition qui les comprend en une quantité comprise entre 0,01 % et 20 % en poids par rapport au poids total de la composition qui les comprend, plus particulièrement entre 0,05 % et 10 %, plus préférentiellement entre 0,1 % et 5 % en poids par rapport au poids total de la composition qui les comprend.

7. Procédé selon une quelconque des revendications précédentes, dans lequel b) le ou les polysaccharides comportant un ou plusieurs groupes amine sont dans une composition cosmétique ayant un pH acide inférieur ou égal à 6, plus particulièrement ayant un pH compris inclusivement entre 2 et 6, plus particulièrement ayant un pH compris inclusivement entre 2,5 et 5,5, plus préférentiellement entre 3,5 et 5, tel que 4,5 ; particulièrement le ou les polysaccharides comportant un ou plusieurs groupes amine b) sont en la présence d'un ou plusieurs acides organiques tels que définis dans la revendication 5.

8. Procédé selon une quelconque des revendications précédentes, dans lequel b) le ou les polysaccharides comportant un ou plusieurs groupes amine ont un poids moléculaire moyen MW inférieur ou égal à 400 kDa, particulièrement inférieur à 200 kDa, plus particulièrement inférieur à 100 kDa, préférentiellement inférieur à 40 kDa, plus préférentiellement compris inclusivement entre 1 kDa et 30 kDa, plus préférentiellement encore ont un MW compris inclusivement entre 3 kDa et 28 kDa.

9. Procédé selon une quelconque des revendications précédentes, dans lequel les étapes (i) et (ii) sont réalisées simultanément ou par application, sur les fibres kératiniques, d'une composition comprenant un ou plusieurs monosaccharides comportant un groupe amine a) tels que définis dans l'une quelconque des revendications 1 à 4 ; et un ou plusieurs polysaccharides comportant un ou plusieurs groupes amine b) tels que définis dans l'une quelconque des revendications 1, 5 à 8, et éventuellement un ou plusieurs acides organiques ou minéraux c).

10. Procédé selon une quelconque des revendications précédentes, dans lequel les étapes (i) et (ii) sont réalisées simultanément ou par application, sur les fibres kératiniques, d'une composition choisie parmi :
- la composition (C') ayant un pH acide qui comprend :
a) un ou plusieurs monosaccharides salifiés comportant un groupe amine tels que définis dans l'une quelconque des revendications 1 à 4 ;
b) un ou plusieurs polysaccharides salifiés comportant un ou plusieurs groupes amine tels que définis dans l'une quelconque des revendications 1, 5 à 8 ; - la composition (C") ayant un pH acide qui comprend :
a) un ou plusieurs monosaccharides non salifiés comportant un groupe amine tels que définis dans l'une quelconque des revendications 1 à 4 ; et
b) un ou plusieurs polysaccharides salifiés comportant un ou plusieurs groupes amine tels que définis dans l'une quelconque des revendications 1, 5 à 8 ; - la composition (C‴) présentant un pH acide qui comprend :
a) un ou plusieurs monosaccharides salifiés comportant un groupe amine tels que définis dans l'une quelconque des revendications 1 à 4 ; et
b) un ou plusieurs polysaccharides non-salifiés comportant un ou plusieurs groupes amine tels que définis dans l'une quelconque des revendications 1, 5 à 8 ; et - la composition (Cʺʺ) ayant un pH acide qui comprend :
a) un ou plusieurs monosaccharides non salifiés comportant un groupe amine tels que définis dans l'une quelconque des revendications 1 à 4 ;
b) un ou plusieurs polysaccharides non salifiés comportant un ou plusieurs groupes amine tels que définis dans l'une quelconque des revendications 1, 5 à 8 ; et
c) un ou plusieurs acides organiques ou minéraux.

11. Procédé selon une quelconque des revendications 1 à 8, qui comprend :
(i) une première étape d'application, sur lesdites fibres, a) d'un ou plusieurs monosaccharides comportant un groupe amine selon l'une quelconque des revendications 1 à 4 ; puis
(ii) une deuxième étape d'application, sur lesdites fibres, b) d'un ou plusieurs polysaccharides comportant un ou plusieurs groupes amine tels que définis dans l'une quelconque des revendications 1, 5 à 8 ; puis
(iii) une étape de traitement thermique à une température supérieure ou égale à 80 °C, en particulier à une température comprise entre 100 °C et 250 °C, de préférence avec un fer à cheveux ;
étant entendu que le ou les ingrédients a) tels que définis précédemment et le ou les ingrédients b) tels que définis précédemment sont présents dans des compositions cosmétiques distinctes.

12. Procédé selon l'une quelconque des revendications précédentes, dans lequel l'étape de traitement thermique ou l'étape de chauffage desdites fibres (iii) est effectuée à une température d'au moins 100 °C, en particulier à une température comprise inclusivement entre 100 °C et 250 °C ; de préférence, l'étape de chauffage des fibres kératiniques est effectuée à une température allant de 150 °C à 220 °C, de préférence allant de 160 °C à 220 °C, préférentiellement allant de 160 °C à 200 °C, notamment allant de 130 °C à 190 °C.

13. Procédé selon une quelconque des revendications précédentes, qui ne met pas en œuvre de polysaccharide oxydé ; plus généralement qui ne met pas en œuvre de composé comprenant au moins un groupe aldéhyde.

14. Procédé selon l'une quelconque des revendications précédentes, dans lequel une étape (ii') de séchage des fibres kératiniques, en particulier des fibres humaines telles que les cheveux, est réalisée avant l'étape (iii) de traitement thermique, ledit séchage est notamment réalisé au moyen d'un sèche-cheveux, d'un casque, ou bien avec une serviette, un absorbant ou par séchage naturel, l'étape de séchage est avantageusement réalisée à une température allant de 20 °C à 70 °C.

15. Composition d'acide (C) c'est-à-dire qui possède un pH inférieur à 7 en particulier inférieur ou égal à 6, plus particulièrement à un pH compris inclusivement entre 2 et 6, préférentiellement à un pH compris entre 2,5 et 5,5, plus préférentiellement entre 3,5 et 5 tel que 4,5 ; et qui comprend :
soit
a) un ou plusieurs monosaccharides salifiés comportant un groupe amine tels que définis dans l'une quelconque des revendications 1 à 4 ;
b) un ou plusieurs polysaccharides salifiés comportant un ou plusieurs groupes amine tels que définis dans l'une quelconque des revendications 1, 5 à 8 ;
et éventuellement c) un ou plusieurs acides minéraux ou organiques de préférence différents des acides suivants : acide aspartique, acide glutamique, acide gluconique, acide pyrrolidonecarboxylique, acide stéarique éthoxylé par 100 OE et 500 OE, acide linoléique, acide succinique et acide alginique ;
soit
a) un ou plusieurs monosaccharides non salifiés comportant un groupe amine tels que définis dans l'une quelconque des revendications 1 à 4 ;
b) un ou plusieurs polysaccharides salifiés comportant un ou plusieurs groupes amine tels que définis dans l'une quelconque des revendications 1, 5 à 8 ;
et éventuellement c) un ou plusieurs acides minéraux ou organiques de préférence différents des acides suivants : acide aspartique, acide glutamique, acide gluconique, acide pyrrolidonecarboxylique, acide stéarique éthoxylé par 100 OE et 500 OE, acide linoléique, acide succinique et acide alginique ;
soit
a) un ou plusieurs monosaccharides salifiés comportant un groupe amine tels que définis dans l'une quelconque des revendications 1 à 4 ;
b) un ou plusieurs polysaccharides non salifiés comportant un ou plusieurs groupes amine tels que définis dans l'une quelconque des revendications 1, 5 à 8 ;
et c) un ou plusieurs acides minéraux ou organiques différents des acides suivants : acide aspartique, acide glutamique, acide gluconique, acide pyrrolidonecarboxylique, acide stéarique éthoxylé par 100 OE et 500 OE, acide linoléique, acide succinique et acide alginique ;
soit
a) un ou plusieurs monosaccharides non salifiés comportant un groupe amine tels que définis dans l'une quelconque des revendications 1 à 4 ;
b) un ou plusieurs polysaccharides non salifiés comportant un ou plusieurs groupes amine tels que définis dans l'une quelconque des revendications 1, 5 à 8 ; et
c) un ou plusieurs acides minéraux ou organiques différents des acides suivants : acide aspartique, acide glutamique, acide gluconique, acide pyrrolidonecarboxylique, acide stéarique éthoxylé par 100 OE et 500 OE, acide linoléique, et acide succinique et acide alginique.

16. Composition telle que définie précédemment qui ne contient pas de polysaccharide oxydé ; plus particulièrement la composition ne comprend pas de composé comprenant au moins un groupe aldéhyde.

17. Utilisation a) d'un ou plusieurs monosaccharides comportant un groupe amine tels que définis dans l'une quelconque des revendications 1 à 4 dans lequel a) le ou les monosaccharides comportant un groupe amine sont dans une composition cosmétique ayant un pH acide, c'est-à-dire un pH inférieur à 7 ; et b) d'un ou plusieurs polysaccharides comportant un ou plusieurs groupes amine tels que définis dans l'une quelconque des revendications 1, 5 à 8 dans lequel b) le ou les polysaccharides comportant un ou plusieurs groupes amine sont dans une composition cosmétique ayant un pH acide c'est-à-dire pH inférieur à 7, pour améliorer l'effet cosmétique des fibres kératiniques de façon intrinsèque, pour empêcher ou diminuer la casse des fibres kératiniques naturelles et/ou pour réparer les fibres kératiniques abimées, tout en gardant ou même en améliorant la maniabilité, les propriétés anti-frisottis et/ou la rémanence du coiffage desdites fibres, en particulier des fibres humaines telles que les cheveux, lorsqu'une étape (iii) de chauffage des fibres est mise en œuvre à au moins 80 °C, en particulier à une température comprise entre 100 °C et 250 °C, de préférence avec un fer à cheveux.

18. Kit à plusieurs compartiments comprenant :
• une première composition cosmétique comprenant a) un ou plusieurs monosaccharides comportant un groupe amine tels que définis dans l'une quelconque des revendications 1 à 4, et éventuellement c) un ou plusieurs acides organiques ou minéraux, dans lequel la composition présente un pH acide, c'est-à-dire inférieur à 7 ;
• une deuxième composition cosmétique comprenant b) un ou plusieurs polysaccharides comportant un ou plusieurs groupes amine tels que définis dans l'une quelconque des revendications 1, 5 à 8, et éventuellement c) un ou plusieurs acides organiques ou minéraux, dans lequel la composition présente un pH acide, c'est-à-dire inférieur à 7 ; et
• un dispositif pour chauffer les fibres kératiniques à une température d'au moins 80 °C, de préférence à une température d'au moins 100 °C, en particulier à une température comprise inclusivement entre 100 °C et 250 °C ; de préférence, l'étape de chauffage des fibres kératiniques est effectuée à une température allant de 150 °C à 220 °C, de préférence allant de 160 °C à 220 °C, préférentiellement allant de 160 °C à 200 °C, notamment allant de 130 °C à 190 °C;
les premières et deuxièmes compositions étant conditionnées chacune dans un ensemble de conditionnement distinct.
